(19) Europäisches Patentamt
European Patent Office
Office européen des brevets

(11) EP 1 608 751 B1

(12) # FASCICULE DE BREVET EUROPEEN

(45) Date de publication et mention de la délivrance du brevet:
**24.11.2010 Bulletin 2010/47**

(21) Numéro de dépôt: **04742352.0**

(22) Date de dépôt: **25.03.2004**

(51) Int Cl.:
***C12N 15/10*** *(2006.01)* ***C12N 9/10*** *(2006.01)*
***A61K 38/45*** *(2006.01)*

(86) Numéro de dépôt international:
**PCT/FR2004/000744**

(87) Numéro de publication internationale:
**WO 2004/087918 (14.10.2004 Gazette 2004/42)**

(54) **PROCEDE DE MODIFICATION IN VIVO DE L'ACTIVITE DE SYNTHESE D'UN METABOLITE PAR MODIFICATION D'UN GENE DONT L'ACTIVITE N'EST PAS L'ACTIVITE ORIGINELLE**

VERFAHREN ZUR IN-VIVO-MODIFIKATION DER SYNTHESEAKTIVITÄT EINES METABOLITEN MITTELS MODIFIKATION EINES GENS, DESSEN AKTIVITÄT NICHT DIE URSPRÜNGLICHE AKTIVITÄT IST

METHOD FOR THE IN VIVO MODIFICATION OF THE SYNTHESIS ACTIVITY OF A METABOLITE BY MEANS OF THE MODIFICATION OF A GENE THE ACTIVITY OF WHICH IS NOT THE ORIGINAL ACTIVITY

(84) Etats contractants désignés:
**AT BE BG CH CY CZ DE DK EE ES FI FR GB GR HU IE IT LI LU MC NL PL PT RO SE SI SK TR**

(30) Priorité: **28.03.2003 FR 0303910**

(43) Date de publication de la demande:
**28.12.2005 Bulletin 2005/52**

(73) Titulaires:
- **INSTITUT PASTEUR
75015 Paris (FR)**
- **CENTRE NATIONAL DE LA RECHERCHE SCIENTIFIQUE (CNRS)
75016 Paris (FR)**

(72) Inventeurs:
- **KAMINSKI, Pierre-Alexandre
F-75003 Paris (FR)**
- **MARLIERE, Philippe
75012 Paris (FR)**

(74) Mandataire: **Caen, Thierry Alain
Santarelli
14 avenue de la Grande Armée
B.P. 237
75822 Paris Cedex 17 (FR)**

(56) Documents cités:
**WO-A-02/083892 WO-A-03/025163**

- **WANG XING-GUO ET AL: "Conversion of a glutamate dehydrogenase into methionine/norleucine dehydrogenase by site-directed mutagenesis" EUROPEAN JOURNAL OF BIOCHEMISTRY, vol. 268, no. 22, novembre 2001 (2001-11), pages 5791-5799, XP002262490 ISSN: 0014-2956**
- **DATABASE BIOSIS [en ligne] BIOSCIENCES INFORMATION SERVICE, PHILADELPHIA, PA, US; 2003 CHANG H K ET AL: "Directed evolution of Comamonas testosteroni GZ39 m-hydroxybenzoate hydroxylase for the synthesis of 4-substituted catechols." Database accession no. PREV200300546363 XP002297541 & ABSTRACTS OF THE GENERAL MEETING OF THE AMERICAN SOCIETY FOR MICROBIOLOGY, vol. 103, 2003, pages O-114 URL, 103RD AMERICAN SOCIETY FOR MICROBIOLOGY GENERAL MEETING; WASHINGTON, DC, USA; MAY 18-22, 2003 ISSN: 1060-2011**
- **WAN LIANGLU ET AL: "In vitro evolution of horse heart myoglobin to increase peroxidase activity" PROCEEDINGS OF THE NATIONAL ACADEMY OF SCIENCES OF THE UNITED STATES OF AMERICA, vol. 95, no. 22, 27 octobre 1998 (1998-10-27), pages 12825-12831, XP002297538 ISSN: 0027-8424**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 608 751 B1

- **DATABASE EMBASE [en ligne] ELSEVIER SCIENCE PUBLISHERS, AMSTERDAM, NL; 1991 CHEN K ET AL: "Enzyme engineering for nonaqueous solvents: Random mutagenesis to enhance activity of subtilisin E in polar organic media" Database accession no. EMB-1992050285 XP002297542 & BIO/TECHNOLOGY 1991 UNITED STATES, vol. 9, no. 11, 1991, pages 1073-1077, ISSN: 0733-222X**
- **CARSON D A ET AL: "SYNTHESIS OF 2 , 3 -DIDEOXYNUCLEOSIDES BY ENZYMATIC TRANS- GLYCOSYLATION" BIOCHEMICAL AND BIOPHYSICAL RESEARCH COMMUNICATIONS, ACADEMIC PRESS INC. ORLANDO, FL, US, vol. 155, no. 2, 15 septembre 1988 (1988-09-15), pages 829-834, XP002044161 ISSN: 0006-291X**
- **HUANG M-C ET AL: "ANALOGS OF 2'-DEOXYADENOSINE: FACILE ENZYMATIC PREPARATION AND GROWTH INHIBITORY EFFECTS ON HUMAN CELL LINES" BIOCHEMICAL PHARMACOLOGY, vol. 30, no. 19, 1981, pages 2663-2671, XP002297539 ISSN: 0006-2952**
- **FREEMAN G A ET AL: "2-amino-9-(3-azido-2,3-dideoxy-beta-D-ery thro-pentofuranosyl)-6-subst ituted-9H-purines: synthesis and anti-HIV activity." BIOORGANIC & MEDICINAL CHEMISTRY. APR 1995, vol. 3, no. 4, avril 1995 (1995-04), pages 447-458, XP002297540 ISSN: 0968-0896**
- **SHORT STEVEN A ET AL: "Active site amino acids that participate in the catalytic mechanism of nucleoside 2'-deoxyribosyltransferase" JOURNAL OF BIOLOGICAL CHEMISTRY, vol. 271, no. 9, 1996, pages 4978-4987, XP002262491 ISSN: 0021-9258**

## Description

La présente invention se rapporte à un procédé d'évolution artificielle *in vitro* et *in vivo* de protéines, ledit procédé permettant de modifier l'activité d'une protéine X de sorte à obtenir un mutant capable d'agir sur un analogue du substrat naturel. Un mutant de la N-désoxyribosyltransférase (DTP) a été obtenu, ladite mutation conférant l'acquisition de l'activité N-didésoxyribosyltransférase.

De manière générale, l'obtention d'enzymes présentant des activités modifiées par rapport à leur activité naturelle est un enjeu important puisque cela permettrait d'avoir accès à des outils puissants dans de nombreuses applications industrielles, notamment en biotechnologie.

Diverses solutions pour effectuer des mutations dirigées dans une molécule d'ADN ont été décrites dans l'état de la technique. Ces techniques consistent à introduire *in vitro* une mutation, une délétion ou une insertion en un site déterminé dans une molécule d'ADN par exemple en utilisant la PCR. Ces diverses techniques sont décrites dans Hall, et al. Protein Eng. 4:601 (1991); Hemsley, et al. Nucleic Acids Research 17:6545-6551 (1989); Ho, et al. Gene 77:51-59 (1989); Hultman, et al. Nucleic Acids Research 18:5107-5112 (1990); Jones, et al. Nature 344:793-794 (1990); Jones, et al. Biotechniques 12:528-533 (1992); Landt, et al. Gene 96:125-128 (1990); Nassal, et al. Nucleic Acids Research 18:3077-3078 (1990); Nelson, et al. Analytical Biochemistry 180:147-151 (1989); Vallette, et al. Nucleic Acids Research 17:723-733 (1989); Watkins, et al. Biotechniques 15:700-704 (1993); Weiner, et al. Gene 126:35-41 (1993). Yao, et al. PCR Methods and Applications 1:205-207 (1992) et dans Weiner et al, Gene 151:1/9-123(1994).

Cependant, il est impossible de savoir à l'avance quel sera l'effet d'une mutation donnée sur l'activité d'une protéine avec de telles techniques.

D'autres méthodes consistent à introduire des mutations au hasard dans le génome par l'emploi d'agents mutagènes (2-aminopurine, hydroxylamine ou ACRIDINE) et à sélectionner les cellules ou organismes montrant le phénotype recherché. Néanmoins, ces méthodes conduisent à l'introduction de nombreuses mutations, parfois létales, et ne sont pas adaptées à faire évoluer une protéine donnée dans un but précis.

Pour répondre aux besoins et problèmes évoqués précédemment, la présente invention propose un procédé pour modifier l'activité d'une protéine combinant des étapes *in vitro* et *in vivo.*

On a trouvé dans le cadre de l'invention que l'introduction de mutations dans la protéine, suivie d'une confrontation avec un analogue du substrat naturel au sein du criblage sélectif permet d'obtenir un protéine mutée ayant une forte activité sur le nouveau substrat. En répétant ces opérations, il est possible d'obtenir des enzymes présentant une activité sur des substrats de plus en plus éloigné du substrat naturel initiale.

Ce procédé est particulièrement adapté à des enzymes du type N-désoxyribosyltransférase (DTP).

L'art antérieur divulgue des techniques de mutagénèse par évolution dirigée portant sur des kinases (WO 02/083892) ou d'autres enzymes dont on cherche à modifier l'activité (WO 03/025163)(Chang, H.K. et al. Directed Evolution of Comamonas testosterone GZ39 m-hydroxybenzoate hydroxylase for the synthesis of 4-substituted catechols (2003) Abstracts of the General Meeting of the American Society for Microbiology, ISSN 1060-2011)(Wan L. et al. In vitro evolution of horse heart myoglobin to increase peroxydase activity (1998) PNAS 95(22):12825-12831). Cependant cet art antérieur ne traite pas spécifiquement des enzymes N-désoxyribosyltransférases dont on souhaiterait qu'elles acquièrent une activité élargie, notamment une activité N-didésoxyribosyltransférase.

Les didésoxynucléosides tels DDI et DDC et leurs dérivés sont les inhibiteurs les plus efficaces connus à ce jour utilisés dans la thérapie contre le virus HIV. Ces composés sont synthétisés chimiquement, mais peuvent également être synthétisés enzymatiquement. La N-désoxyribosyltransférase de *Lactobacillus leichmannii* (ainsi que celle de *L. helveticus*) enzyme qui catalyse le transfert de désoxyribose entre deux bases puriques ou pyrimidiques est également capable de transférer le 2',3'-didésoxyribose entre ces mêmes bases (Carson et Wasson, 1988). Ainsi, plusieurs pyrazolo (3,4-d) pyrimidine et triazolo (4,5-d) pyrimidine dérivés de la 2',3'-didésoxycytidine et de la base correspondante ont été synthétisés (Fischer et coll, 1990). La réaction de transfert de 2',3'-didésoxyribose est toutefois nettement moins efficace que celle effectuée avec le 2'-désoxyribose. Dans le but de disposer d'une enzyme ayant une activité spécifique plus élevée que l'enzyme native pour le transfert de 2',3 -didésoxyribose, nous avons combiné une étape de mutagenèse aléatoire du gène *ntd* de *L. leichmannii* à une étape de sélection des mutants en utilisant un crible génétique.

Un crible fonctionnel permettant de sélectionner la production d'uracile a été établi chez *E. coli,* en délétant le gène *pyrC* qui commande la conversion de carbamyl aspartate en dihydroorotate ainsi que les gènes *codA* et *cdd* qui commandent respectivement la désamination de la cytosine et de la (désoxy)cytidine pour donner la souche PAK 9. La souche PAK 9 présente une exigence en uracile (u) qui ne pourra être satisfaite par l'apport d'uridine (R-U), de désoxyuracile (dR-U) ou de didésoxyuracile (ddR-U). L'utilisation de didésoxyuracile (ddR-U) pourra cependant être sélectionnée dans la souche PAK9 si un variant de la N-désoxyribosyltransférase est capable de réaliser une des deux réactions suivantes:

i) ddR-U $\rightarrow$ U + ddR,

ii) ddR-U + C ⇄ ddR-C + U.

Une mutagénèse aléatoire du gène *ntd* de *L. leichmannii* a été effectuée, les produits mutés ont ensuite été clonés dans un plasmide et le mélange utilisé pour transformer la souche PAK9. Les clones transformants ont été sélectionnés en milieu minéral glucose additionné de didésoxyuracile (ddR-U) et de cytosine (C). Plusieurs transformants ont été obtenus et sont capables de réaliser l'échange

ddR-Pyr + Pur ⇄ ddR-Pur + Pyr

ainsi que

dR-Pyr + Pur ⇄ dR-Pur + Pyr.

Les séquences nucléotidiques des différents variants de NTD sont identiques et ne diffèrent du gène sauvage que d'une mutation. Leurs activités enzymatiques ont été évaluées à partir d'extraits bruts ou des protéines purifiées. L'activité spécifique de NTD* est 10 fois inférieure à celle de NTD pour le transfert de désoxyribose mais est 7 fois supérieure pour le transfert de didésoxyribose.

L'enzyme sélectionnée trouve son application dans la synthèse enzymatique de 2,3'-didésoxynucléoides de bases naturelles ou modifiées (5-halogéno-pyrimidines), comportant ou non des radioéléments. Le procédé peut être étendu à la sélection de variants capables de transférer, des dérivés de 2'-désoxyribose ou 2',3'-didésoxyribose entre bases (tel que, 3'-amino-2',3'-didésoxyribose ou 3'-azido-2',3'-didésoxyribose).

En outre, dans le procédé selon l'invention, on peut utiliser des cellules dans lesquelles une voie métabolique a été inactivée. Le crible sélectif consiste à complémenter cette déficience en produisant le produit P pour lequel les cellules sont auxotrophes à partir d'un analogue du substrat naturel de la protéine X.

Alternativement, on peut faire évoluer une protéine X par complémentation d'une protéine apparentée Y, X et Y appartenant tous deux à la même classe de la nomenclature enzymatique EC ou à des classes voisines.

## Description

Ainsi, de manière générale, la présente invention se rapporte à un procédé d'évolution artificielle *in vitro* et *in vivo* de protéines, ledit procédé permettant de faire évoluer *in vivo* une protéine X par complémentation soit d'une protéine apparentée, soit par complémentation d'une voie métabolique inactivée.

Un tel procédé permet de faire évoluer une protéine X de sorte à en modifier ses caractéristiques et comprend les étapes suivantes :

a) obtention de mutants X* de la séquence codant pour la protéine X par mutagénèse aléatoire ;
b) transformation de cellules comportant un phénotype [P-] avec des vecteurs comportant les acides nucléiques mutés obtenus à l'étape a) codant pour les protéines X*, P- signifiant que lesdites cellules sont auxothrophes pour la substance P, P étant le produit de l'action de X sur son substrat naturel S ;
c) mise en culture desdites cellules dans un milieu comprenant un substrat S*, S* étant un analogue du substrat naturel S de la protéine X ;
d) sélection des cellules [P- :: X*] qui ont survécu à l'étape c) dans lesquelles les protéines X* sont capables de réaliser la biosynthèse du produit P à partir du substrat S*.

La protéine mutante X obtenue est une protéine possédant une activité voisine de la protéine naturelle X, X* et X appartenant à des classes enzymatiques communes ou voisines présentant au moins les trois premiers chiffres des classes EC de la nomenclature internationale à 4 chiffres. Pour le passage d'une classe à une autre, on peut répéter le procédé mentionné ci-dessus avec à chaque passage l'addition d'une modification supplémentaire sur l'analogue de substrat désigné par S*. Par analogue de substrat, on entend le substrat S naturel de la protéine X naturelle comportant une modification ou une altération. Par modification de ce substrat, on entend l'addition ou la suppression d'au moins un atome, un groupe ou substituant, la modification de la conformation spaciale du substrat (isomère, enantiomère, diastéroisomère). Cette modification peut être minime ou importante du point de vue structurel. Dans le cas, où on cherche à modifier de manière substantielle l'activité de la protéine (ou enzyme), on peut procéder par répétition du procédé en modifiant d'avantage le substrat S* à chaque nouveau cycle de sélection. Peu à peu, la protéine accumule des mutations qui sont responsables de la modification de son activité.

Dans ce procédé, les cellules utilisées à l'étape b) sont obtenues par inactivation d'un moins un gène impliqué dans la voie métabolique naturelle conduisant au produit P.

Ainsi, la protéine X* obtenue complémente la déficience de la voie métabolique naturelle conduisant au produit

P dans un milieu pourvu du substrat S*.

Par "complément", on entend la suppression du phénotype auxotrophe résultant de l'inactivation du gène ou de la voie métabolique.

Alternativement, les cellules peuvent être des cellules dans lesquelles le gène codant pour une protéine apparentée à X a été inactivé au préalable.

Par "inactivation", on entend une délétion en tout ou en partie, une insertion, ou une mutation rendant inopérant le gène. L'inactivation peut également consister en une modification conduisant à un phénotype du type Ts (température sensible). Dans ce cas, les cellules sont cultivées à des températures non permissibles pendant la phase de sélection (étapes c) et d)).

De préférence, la protéine apparentée possède au moins les trois premiers chiffres de la nomenclature internationale EC à 4 chiffres.

Avantageusement, l'activité de la protéine X sur le substrat S est au moins 2, 5, 10, 25, 50, 100 ou 1000 fois supérieure à son activité sur le substrat S*. Parallèlement, l'activité de la protéine X* sur le substrat S* est au moins 5, 10, 25, 50, 100 ou 1000 fois supérieure à son activité sur le substrat S.

Parmi les protéines visées, on peut citer :

**Numéro EC Nom selon la nomenclature internationale**

**- les ribosyltransférases, telles que par exemple :**

| | |
|---|---|
| 2.4.2.5 | Nucleoside ribosyltransferase. |
| 2.4.2.6 | Nucleoside deoxyribosyltransferase. |
| 2.4.2.7 | Adenine phosphoribosyltransferase |
| 2.4.2.8 | Hypoxanthine phosphoribosyltransferase. |
| 2.4.2.9 | Uracil phosphoribosyltransferase. |
| 2.4.2.10 | Orotate phosphoribosyltransferase. |
| 2.4.2.11 | Nicotinate phosphoribosyltransferase. |
| 2.4.2.12 | Nicotinamide phosphoribosyltransferase. |
| 2.4.2.14 | Amidophosphoribosyltransferase. |
| 2.4.2.17 | ATP phosphoribosyltransferase. |
| 2.4.2.18 | Anthranilate phosphoribosyltransferase. |
| 2.4.2.20 | Dioxotetrahydropyrimidine phosphoribosyltransferase. |
| 2.4.2.21 | Nicotinate-nucleotide-dimethylbenzimidazole phosphoribosyltransferase. |
| 2.4.2.22 | Xanthine-guanine phosphoribosyltransferase. |
| 2.4.2.29 | Queuine tRNA-ribosyltransferase. |
| 2.4.2.30 | NAD(+) ADP-ribosyltransferase. |
| 2.4.2.31 | NAD(P)(+)--arginine ADP-ribosyltransferase. |
| 2.4.2.36 | NAD(+)--diphthamide ADP-ribosyltransferase. |
| 2.4.2.37 | NAD(+)--dinitrogeN-reductase ADP-D-ribosyltransferase. |

**- les kinases, telles que par exemple :**

| | |
|---|---|
| 2.7.1.20 | Adenosine kinase. |
| 2.7.1.21 | Thymidine kinase. |
| 2.7.1.38 | Phosphorylase kinase. |
| 2.7.1.49 | Hydroxymethylpyrimidine kinase. |
| 2.7.1.74 | désoxycytidine kinase (DCK). |
| 2.7.4.6 | Nucleoside-diphosphate kinase. |
| 2.7.4.7 | Phosphomethylpyrimidine kinase. |
| 2.7.4.8 | Guanylate kinase. |
| 2.7.4.9 | Thymidylate kinase. |
| 2.7.4.10 | Nucleoside-triphosphate--adenylate kinase. |
| 2.7.4.11 | (Deoxy)adenylate kinase. |

(suite)

| **- les kinases, telles que par exemple :** | |
|---|---|
| 2.7.4.12 | T2-induced deoxynucleotide kinase. |
| 2.7.4.13 | (Deoxy)nucleoside-phosphate kinase. |

| **- les nucléotidyl transférases, telles que par exemple :** | |
|---|---|
| 2.7.7.6 | DNA-directed RNA polymerase. |
| 2.7.7.7 | DNA-directed DNA polymerase. |
| 2.7.7.8 | Polyribonucleotide nucleotidyltransferase. |
| 2.7.7.19 | Polynucleotide adenylyltransferase. |
| 2.7.7.25 | tRNA adenylyltransferase. |
| 2.7.7.48 | RNA-directed RNA polymerase. |
| 2.7.7.49 | RNA-directed DNA polymerase. |
| 2.7.7.50 | mRNA guanylyltransferase. |

| **- les phosphorylases, telles que par exemple** | |
|---|---|
| 2.4.2.1 | Purine-nucleoside phosphorylase. |
| 2.4.2.2 | Pyrimidine-nucleoside phosphorylase. |
| 2.4.2.3 | Uridine phosphorylase. |
| 2.4.2.4 | Thymidine phosphorylase. |
| 2.4.2.7 | Adenine phosphoribosyltransferase. |
| 2.4.2.8 | Hypoxanthine phosphoribosyltransferase. |
| 2.4.2.9 | Uracil phosphoribosyltransferase. |
| 2.4.2.15 | Guanosine phosphorylase. |
| 2.4.2.23 | Deoxyuridine phosphorylase. |
| 2.4.2.28 | 5'-methylthioadenosine phosphorylase. |

De préférence, la protéine X est sélectionnée parmi les ribosyltransférases appartenant aux classes EC 2.4.2.-, en particulier les N-déoxyribosyltransférases de la classe EC 2.4.2.6.

Bien entendu, d'autres enzymes, notamment les enzymes du métabolisme ou du catabolisme, peuvent faire l'objet d'une modification grâce au procédé selon l'invention. Ces enzymes et leur numéro EC respectif sont répertoriés par le Committee of the International Union of Biochemistry and Molecular Biology (IUBMB) à l'adresse suivante : http: //expasy.proteome.org.au/enzyme/

La mutagénèse aléatoire de l'étape a) peut être effectuée soit par variation de la concentration en manganèse lors de la réaction PCR, soit par utilisation d'analogues de nucléotides promutagènes ou encore par la mise en oeuvre d'amorces comprenant une séquence aléatoire. Différentes techniques sont décrites dans les documents US 6,323,030 (Methods for generating polynucleotides having desired characteristics by iterative selection and recombination), US 6,177,263 (Recombination of polynucleotide sequences using random or defined primers), WO 01/66798 (Random truncation and amplification of nucleic acid), and EP1205547 (DNA mutagenesis by random fragmentation and reassembly).

Les cellules utilisées dans le cadre de l'invention sont des cellules procaryotes ou eucaryotes, de préférence *E. coli.*

Dans un mode de réalisation particulier, l'invention vise un procédé tel que décrit ci-dessus pour faire évoluer une N-déoxyribosyltransférase (DTP) de sorte à obtenir une N-didéoxyribosyltransférase **caractérisé en ce qu**'il comprend les étapes suivantes :

a) obtention de mutants DTP* de la séquence codant pour une N-déoxyribosyltransférase (DTP) par mutagénèse aléatoire ;
b) transformation de cellules comportant un phénotype [N-] avec des vecteurs comportant les acides nucléiques mutés obtenus à l'étape a) codant pour les protéines DTP*, N- signifiant que lesdites cellules sont auxothrophes pour au moins un nucléoside, ledit nucléoside étant le produit de l'action de DTP sur son substrat naturel dR-N ;
c) mise en culture desdites cellules dans un milieu comprenant un substrat ddR-N ;

d) sélection des cellules [N-:: DTP*] qui ont survécu à l'étape c) dans lesquelles les protéines DTP* sont capables de réaliser le transfert du didéoxyribose (ddR) d'un didéoxyribonucléoside à un autre nucléoside conduisant à la production du nucléoside N nécessaire pour la survie des cellules.

Par nucléoside N, on entend un nucléoside naturel, c'est à dire des molécules constituées d'un sucre relié à une base hétérocyclique par une liaison N-glycosidique, les bases étant des pyrimidines (thymine, uracile, cytosine) ou des purines (adénine, guanine parmi les bases usuelles). Par N- , on entend un phénotype [A-, T-, G-, C-, U- ou I-].

L'enzyme NTD* obtenu peut être capable de reconnaître et transférer un analogue de déoxyribose tel que le didéoxyribose, mais également d'agir sur des analogues de nucléoside. Ainsi, l'analogue de substrat S* utilisé peut être un analogue de déoxyribonucléoside comportant au moins une modification chimique sur la base et/ou sur le ribose.

Plus particulièrement, la N-déoxyribosyltransférase (DTP) est la DTP de *Lactobacillus leichmannii* de SEQ ID No 1.

Dans ce procédé, on peut utiliser à l'étape b) des bactéries *E. coli ΔpyrC, Δcod A, Δcdd* déficientes dans la voie métabolique conduisant à l'uracile. Cette bactérie est désignée comme étant la souche PAK 9 déposée à la CNCM le 27 Juin 2002 sous le N° I-2902.

Dans un deuxième aspect, l'invention se rapporte à la protéine mutée X* susceptible d'être obtenue à partir du procédé décrit ci-dessus, **caractérisée en ce qu**'elle présente une activité modifiée par rapport à la protéine X initiale.

La protéine X* peut être une N-déoxyribosyltransférase mutée susceptible d'être obtenue à partir du procédé selon l'invention présentant une activité N-didéoxyribosyltransférase.

La présente invention a aussi pour objet une N-désoxyribosyltransférase mutée susceptible d'être obtenue à partir des procédés ci-dessus décrits, **caractérisée en ce qu**'elle présente une activité N-didéoxyribosyltransférase et/ou une activité sur des analogues de déoxy ou didéoxyribonucléoside comportant une base modifiée.

Avantageusement, l'invention vise la N-déoxyribosyltransférase mutée précitée **caractérisée en ce qu**'elle comprend la séquence SEQ ID No 2 comportant la mutation G9S et en ce qu'elle présente une activité N-didéoxyribosyltransférase.

L'invention concerne également un acide nucléique comprenant une séquence codant pour la N-déoxyribosyltransférase mutée (NTD*) mentionnée ci-dessus, notamment la séquence SEQ ID No 3.

L'invention porte également sur un vecteur d'expression comprenant ladite séquence codante. Cette séquence peut être fusionnée à un promoteur efficace dans les cellules eucaryotes et/ou procaryotes. Le vecteur peut être un plasmide capable de transformer et de se maintenir chez *E. coli.* Le vecteur peut se maintenir dans la bactérie de manière stable ou transitoire.

L'invention vise également une cellule hôte comprenant un vecteur tel que décrit précédemment.

Dans un troisième aspect, l'invention se rapporte à l'utilisation d'une N-didéoxyribosyltransférase décrite ci-dessus pour le transfert d'un didéoxyribose (ddR) d'un didéoxyribonucléoside sur un autre nucléoside. Cet enzyme obtenue à partir du procédé selon l'invention est particulièrement utile pour la préparation d'analogues de nucléosides possédant des propriétés antitumorales, notamment du ddl ou ddC.

Ainsi, l'invention porte également sur un procédé de préparation de composés comprenant une étape consistant à mettre en oeuvre une protéine mutée définie ci-dessus.

Ce procédé est particulièrement avantageux pour la préparation d'analogues de nucléosides ou nucléotides utiles pour le traitement du cancer ou de maladies infectieuses, notamment des didéoxyribonucléosides, en particulier le ddC ou ddl.

L'invention a également trait à la souche d'*E. coli* PAK 9 de génotype Δ*pyrC* ::Gm, Δ*codA* ::Km, *cdd* ::*Tn*10 déposée à la CNCM sous le numéro d'accession I-2902.

On se référera aux légendes des figures ci-après pour la suite de la description.

**<u>Légendes</u>**

- Figure 1 : Voies de biosynthèses
- figure 1a) la synthèse *"de novo"* de l'ADN à partir de précurseurs simples.
- figure 1b) la voie de sauvegarde ou de recyclage bien moins coûteuse en énergie et impliquant des réactions de transfert de sucre à partir de bases préformées (issues de la dégradation hydrolytique d'acides aminés et de nucléotides).
- Figure 2 : Cycle catalytique de NTD
- Figure 3 : Réaction ddU+I = ddl+U pour psu-ntdA
- Figure 4 : Réaction ddU+I = ddl+U pour psu-ntd*C
- Figure 5 : Réaction dU+I = dI+U pour pSU-ntdA

- Figure 6 : Réaction dU+I = dI+U pour pSU-ntd*C

**EXEMPLE 1 : Synthèse enzymatique de nucléosides**

La synthèse des nucléosides chez *E. coli* peut se faire selon deux procédés; [Agnete MUNCH-PETERSEN (1983). "Metabolism of nucleotides, nucleosides and nucleobases in microorganisms" published by Academic Press] (voir figures 1a et 1b).

Il existe deux classes d'enzymes qui catalysent le transfert d'un 2-désoxyribosyle vers une base azotée ; voir ci-après et [Jane R. HANRAHAN & David W. HUTCHINSON (1992). "The enzymatic synthesis of antiviral agents". Journal of Biotechnology; vol.23; 193-210. Celles-ci sont parfois employées pour la synthèse d'analogues de nucléosides].

**1.1 Les nucléosides phosphorylases**

Pour revue voir [Thomas A. KRENITSKY, George W. KOASALKA & Joel TUTTLE (1981). "Purine nucleoside synthesis, an efficient method employing nucleoside phosphorylase". Biochemistry; vol. 20; 3165-3621].

La plupart des micro-organismes (tel *E. coli*) utilise cette voie de synthèse qui débute par la phosphorylation "réversible" d'un ribonucléoside en ribose-1-phosphate (ou 2-désoxyribonucléoside en 2-désoxyribose-1-phosphate) à partir de phosphate inorganique avec libération de la base donneuse (1) suivie de l'addition de la base acceptrice (2).

(1) : $(d)R\text{-}Base_1 + H_2PO_4 \rightleftarrows (d)R\text{-}1\text{-}phosphate + Base_1$

(2) : $(d)R\text{-}1\text{-}phosphate + Base_2 \rightleftarrows (d)R\text{-}Base_2 + H_2PO_4$

(3) = (1) + (2) : $(d)R\text{-}Base_1 + Base_2 \rightleftarrows Base_1 + (d)R\text{-}Base_2$

(d)R = (2-désoxy)ribose

[E] = PNPase (purine phosphorylase) ou Deo D pour Base = purine Urd Pase (uridine phosphorylase) ou UDP pour Base = U (et T de façon marginale)

dThd Pase (thymidine phosphorylase) ou Deo A pour Base = T (et U de manière marginale)

Ces enzymes catalysent la même réaction avec des substrats différents.

Pourtant deux classes d'enzymes sont distinguées [A.R. MUSHEGIAN & E.V. KOONIN (1994). "Unexpected sequence similarity between nucleosidases and phosphoribosyltransferases of different specificity". Protein Science ; vol.3 ; 1081-1088] :

- d'une part la famille I à laquelle sont rattachées Deo D et UDP (chez *E. coli* les séquences des acides aminés qui les constituent sont très similaires);
- d'autre part la famille II regroupant les thymidine phosphorylases. Des enzymes ayant la même fonction et pouvant fixer un même substrat (telles UDP et Deo A) n'ont donc pas forcément des séquences d'acides aminés apparentées.

**1.2 Les N-désoxyribosyltransférases (NTD)**

Les N-désoxyribosyltransférases catalysent le clivage des liaisons glycosidiques des 2-désoxynucléotides. Elles sont présentes chez certains micro-organismes qui ne possèdent pas ou peu de purine et de pyrimidine phosphorylase (lactobacilles par exemple) [6-8]. Elles participent au recyclage des nucléotide.

**Réactions catalysées selon le type d'enzymes**

Deux types d'enzyme ont été caractérisés, [José HOLGUIN & Robert CARDINAUD (1975). "Trans-N-Deoxyribosylase: substrate specific studies". European Journal of Biochemmistry; vol.54; 515-520].

**Purine désoxyribosyltransférase ou NTD I :**

Elle catalyse exclusivement le transfert réversible d'un sucre d'une base purique (base donneuse) vers une autre purine (base receveuse).

(1) : $(d)R\text{-}pur_1 + pur_2 \rightleftarrows pur_1 + (d)R\text{-}pur_2$

**Pyrimidine/Purine désoxyribosyltransférase ou NTD II :**

Elle catalyse majoritairement le transfert entre purine et pyrimidine selon les équations réversibles suivantes :

(2) : (d)R-$pyr_1$ + $pyr_2$ ⇄ $pyr_1$ + (d)R-$pyr_2$

(3) : (d)R-$pyr_1$ + $pur_2$ ⇄ $pyr_1$ + (d)R-$pur_2$

**Mécanisme réactionnel (figure 2)**

NTD II de *Lactobacillus delbruckii* réagirait selon un mécanisme "ping-pong-bi-bi" qui ferait intervenir deux substrats et deux produits [José HOLGUIN & Robert CARDINAUD (1975). "Trans-N-Deoxyribosylase: Purification by affinity chromatography and characterisation". European Journal of Biochemmistry; vol.54; 505-514 ; C. DANZIN & Robert CARDINAUD (1974). "Deoxyribosyl transfer catalysis with trans-N-deoxyribosylase. Kinetic studies of purine to purine trans-N-deoxyribosylase. European Journal of Biochemistry; vol. 48; 255-252 ; C. DANZIN & Robert CARDINAUD (1976). "Deoxyribosyl transfer catalysis with trans-N-deoxyribosylase. Kinetic studies of purine (pyrimidine) to purine (pyrimidine) trans-N-deoxyribosylase. European Journal of Biochemistry; vol.62; 356-372].

On suppose que le sucre du nucléoside donneur ($dBase_1$) se lie de façon covalente à l'enzyme. Une réaction intramoléculaire au sein de ce complexe binaire permet le clivage de la liaison β-glycosidique et la formation d'un complexe ternaire E-désoxyribosyl-$Base_1$ suivie de la libération du premier produit ($Base_1$). La base acceptrice ($Base_2$) se fixe alors sur l'intermédiaire binaire et après réaction intramoléculaire sur le site actif de l'enzyme, le second produit ($dBase_2$) est libéré. L'enzyme peut dès lors mener une autre catalyse.

**Propriétés physico-chimiques**

Les deux enzymes ont un poids moléculaire voisin (évalué aux alentours de 100 kDa) mais elles diffèrent par leur stabilité thermique (activité observée jusqu'à 45°C pour NTD 1 et 55°C pour NTD II) et leur pH optimum (6.4 pour NTD I est 6.0 pour NTD II).
Le gène *ntd de Lactobacillus delbruckii* codant pour NTD II d'une longueur de 471 bp code pour la synthèse d'une protéine de 157 acides aminés et de poids moléculaire total de 110 kDa [William J.COOK, Steven A. SHORT & Steven E. EALICK (1990). "Crystallization & preliminary X-ray investigation of recombinant Lactobacillus leichmanii nucleoside 2-deoxyribosyltransferase". The Journal of Biological Chemistry; vol.265; No.5; 2682-2683]. La structure cristalline de l'enzyme NTD II de *L. delbruckii* a été déterminée avec une résolution de 2,5 Å. C'est un hexamère (trimère de dimères) constitué de six sous-unités identiques de 18 kDa. Chaque sous-unité possède au centre un feuillet β parallèle composé de cinq brins de longueurs diverses et entouré par quatre hélices α disposées de façon asymétrique. Chacune comprend un site actif, mais les six centres catalytiques, distants deux à deux d'environ 20 Å, requièrent la participation des chaînes latérales des sous-unités voisines [Shelly R. ARMSTRONG, William J.COOK, Steven A. SHORT & Steven E. EALICK (1996). "Crystal structures of nucleoside 2-deoxyribosyltransferase in native & ligand-bound forms reval architecture of the active site". Structure; vol.4; No.1; 97-107]. Ces dernières sont impliquées dans le positionnement de l'acide aminé catalytique (le glutamate 98) [ David J.T. PORTER, Barbara M. MERRIL & Steven A. SHORT (1995). "Identification of the active site nucleophile nucleoside 2-deoxyribosyltransferase as glutamic acid 98". The Journal of Biological chemistry; vol.270; No.26; 15551-15556].

**Synthèse enzymatique d'analogues de nucléosides**

Les réactions de transfert, hautement stéréospécifiques, produisent en présence d'une transférase NTD I ou NTD II, exclusivement l'anomère β du nucléoside (ce qui évite l'étape de séparation des isomères α et β).

L'enzyme possède une grande spécificité vis-à-vis des 2'-désoxyribonucléotides mais tolère un grand nombre d'analogues modifiés sur le sucre ou sur la base. La thymidine et la cytosine semblent les plus efficaces donneurs de sucre. D'autre part le transfert peut s'effectuer sur un large panel de bases receveuses. Citons par exemple les purines substituées en position 6 [D. BETBEDER, D.W. HUTCHINSON & A.O. RICHARDS (1989). "The stereoselective enzymatic synthesis of 9-β-D-2',3'-dideoxynucleosides of N(6)-substitued purines". Antiviral Chem. Chemother ; vol.17; 4217-4222] et dYTP.

**dYTP:**

L'imidazole-4-carboxamide noté Y a été proposé comme purine simplifiée. Cet analogue a pour formule:

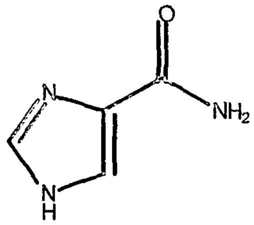

Il a été rapporté que le nucléotide dYTP pouvait se substituer à dATP ou dGTP lors de la copie de l'ADN ce qui introduit des mutations. On peut également citer les composés décrits WO 01/96354 (Institut Pasteur) de formule générale :

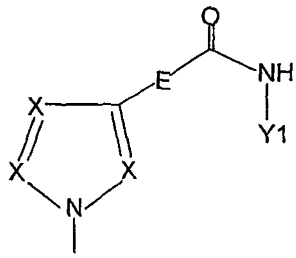

Les enzymes NTD se révèlent capables de catalyser de façon marginale la réaction d'échange entre un 2',3'-didésoxyribose et une base acceptrice :

$$\text{dd-1'-Base}_1 + \text{Base}_2 \rightleftarrows \text{Base}_1 + \text{dd-1'-Base2}$$

dd = 2',3'-didéoxyribose

Néanmoins la vitesse de ce transfert reste très faible comparée à celle caractérisant l'échange de désoxyriboses.

- Les 2',3'-didésoxyribonucléotides présentent un intérêt évident en tant que terminateurs de chaîne dans les procédures de séquençage. De plus la 2',3'-didésoxyadénosine (ddA) et la 2',3'-didésoxyinosine (ddI) sont utilisés à des fins thérapeutiques notamment dans le cas du virus du SIDA: ces analogues inhibent de manière efficace la replication du VIH (virus d'immunodéficience humaine) [H. MITSUYA & S. BRODER (1987). "Strategies for antiviral therapy in AIDS". Nature ; vol. 325 ; 773-778].

A cette fin, l'invention apporte un nouveau procédé d'obtention de mutants de l'enzyme NTD II afin de sélectionner des enzymes mutantes qui ont une plus forte spécificité envers les 2',3'-didésoxyribonucléosides que l'enzyme native.

**EXEMPLE 2 : Application du procédé selon l'invention** pour l'obtention **de NTD***

**MATERIELS ET METHODES**

Les souches d'*E. coli* sont cultivées en milieu Luria-Bertani (LB) ou en milieu minimum MS (Richaud et coll 1993). Les antibiotiques kanamycine, Km, chloramphénicol Cm, sont utilisés à la concentration finale de 25 $\mu$g/ml ; tetracycline, Tc et gentamycine, Gm, 10 $\mu$g/ml. Les nucleosides et bases sont utilisés dans les milieux de culture à la concentration finale de 0,3 mM. Les techniques de biologie moléculaire ont été faites selon Sambrook et coll (1989)

Les produits d'amplification sont purifiés à l'aide du QIAquick PCR purification (QIAgen)

Les fragments d'ADN purifiés sur gel d'agarose sont extraits à l'aide du Kit Jetsorb (Genomed) ou du kit QIAquick gel extraction (QIAgen). L'ADN plasmidique est purifié à l'aide du kit QIAprep spin miniprep (QIAgen)

**1- Construction de la souche PAK9 ( MG1655 ∆pyrC ::Gm, ∆codA ::Km, cdd ::Tn10)**

**• ∆codA ::Km :**

Les oligonucléotides codBL (5'-NNNCCCGGGCTTCTTGCTCGCTTCTCGTTTGG-3') (SEQ ID No 4) et cynTR (5'-NNGGATCCGTTTGACCGTAGCGGGCGAAC-3') (SEQ ID No 5) ont été utilisés pour amplifier à partir de l'ADN de la souche d'*E.coli* MG1655 un fragment de 3,3kb contenant le gène *pyrC.*

Les réactions de PCR sont effectuées dans un volume final de 100 µL comprenant 100 pmol de chaque oligonucléotides, 700 ng d'ADN de la souche MG1655, les dNTPs à la concentration finale de à 200 µM, 10 µL de tampon de réaction 10 fois concentré de la Taq polymérase (Roche) et 5 U de *Taq* polymérase (Roche). Les paramètres d'amplification sont : 1 cycle de 5' à 95°C, 25 cycles comportant chacun les trois étapes suivantes : 30 secondes à 95°C, 30 secondes à 60°C, 2 minutes à 72°C, puis un cycle de 10 minutes à 72°C. Le produit d'amplification a ensuite été digéré par les enzymes de restriction *Xma*I et *Bam*HI, purifié sur gel et ligaturé au plasmide pMTL22 (Chambers et coll. 1988) digéré par les mêmes enzymes et purifié sur gel comme ci-dessus. Le mélange de ligature a été utilisé pour transformer la souche β 2033. L'ADN plasmidique de plusieurs transformants a été préparé et utilisé comme ADN matrice pour déléter le gène *codA* en utilisant les oligonucléotides codBR (5'-NGAATTCTTATTCGACACTGTTAGCCTCC-3') (SEQ ID No 6) et cynTL (5'-NGAATTCACGACTGGGTTACAGCGAGC-3') (SEQ ID No 7) dans les mêmes conditions et avec les mêmes paramètres que ci-dessus. Le produit d'amplification a ensuite été digéré par l'enzyme de restriction *Eco*RI, purifié sur gel d'agarose et ligaturé au fragment *Eco*RI de 1,2 kb de pUC4K (Pharmacia) conférant la résistance à la kanamycine. Le mélange de ligature a été utilisé pour transformer la souche β 2033. L'ADN plasmidique de plusieurs transformants résistants à la kanamycine a été préparé puis utilisé comme ADN matrice pour une réaction d'amplification avec les oligonucléotides codBL et cynTR. Le produit d'amplification a ensuite été purifié sur gel d'agarose, digéré par l'enzyme de restriction *Dpn*I pendant 12 heures à 37°C puis extrait au phénol et précipité à l'éthanol. L'ADN remis en solution dans de l'eau a été utilisé pour transformer la souche MG1655 hébergeant le plasmide pKOBEG par électroporation (don de J-M Ghigo, Unité des membranes bactériennes). Les clones transformants obtenus sur milieu LB en présence de kanamycine ont ensuite été testés pour la sensibilité à la 5-fluorouracile et la résistance à la 5-fluorocytosine. La délétion du gène *codA* a également été vérifiée par amplification avec les oligonucléotides codBL et cynTR.

**• ∆ *pyrC*::Gm :**

Les oligonucléotides ycEL (5'-NNNCCCGGGGCCGACCTGCTGGCCCACTCTGACGG-3') (SEQ ID No 8) et dinR (5'-NNGGATCCCCCGGCGGCAGCGCCTACGGAACCGCTGCC-3') (SEQ ID No 9) ont été utilisés pour amplifier à partir de l'ADN de la souche d'*E.coli* MG1655 un fragment de 3,1 kb contenant le gène *pyrC*. selon le protocole décrit pour *codA.* Le produit d'amplification a ensuite été inséré dans le plasmide pCR2.1-T0P0 (Invitrogen, USA) et le mélange utilisé pour transformer la souche TOP10F' (Invitrogen, USA). L'ADN plasmidique des clones transformants a ensuite été préparé et utilisé comme matrice dans une réaction d'amplification avec les oligonucléotides yceR (5'-NGAATTCTTAATCAGTAAATGGAATGACAATTTCGCC-3') (SEQ ID No 10) et dinL (5'-NGAATTCAAATCGTAGCTTCCTGTTGTCATTAGG-3') (SEQ ID No 11).

Les paramètres d'amplification 1 cycle de 5' à 95°C, 25 cycles comportant chacun les trois étapes suivantes : 30 secondes à 95°C, 30 secondes à 65°C, 3 minutes à 72°C, puis un cycle de 10 minutes à 72°C. Le produit d'amplification a ensuite été digéré par l'enzyme de restriction *Eco*RI purifié sur gel et ligaturé à un fragment *Eco*RI de 1,1 kb conférant la résistance à la gentamycine. Le mélange de ligature a été utilisé pour transformer la souche β 2033. L'ADN plasmidique des clones transformants résistant à la gentamycine a ensuite été préparé et utilisé dans une réaction d'amplification avec les oligonucléotides yceL et dinR. Le produit d'amplification a ensuite été purifié sur gel d'agarose puis digéré par l'enzyme de restriction *Dpn*I pendant 12 heures à 37°C et précipité à l'éthanol après extraction au phénol. L'ADN a été remis en solution dans de l'eau puis utilisé pour électroporer la souche MG1655 hébergeant le plasmide pKOBEG. Les clones transformants obtenus sur milieu LB en présence de gentamycine et d'uracile Les clones auxotrophes pour l'uracile étaient également délétés du gène *pyrC.*

**• ∆*pyrC* ::Gm, ∆*codA* ::Km, *cdd*: :*Tn*10.**

La mutation *codA* ::Km portée par la souche PAK1 a été transduite dans la souche PAK2 (MG1655 ∆*pyrC* :: Gm) à l'aide du phage P1. Les transductants ont été sélectionnés sur milieu LB supplémenté en kanamycine, gentamycine et uracile. De la même manière la mutation *cdd* ::*Tn*10 a été introduite dans la souche PAK15 (MG1655 ∆*pyrC* ::Gm, ∆*codA* ::Km) par transduction à l'aide d'un stock de phage P1 préparé à partir de la souche β7234 ∆deo, *argE* : :*am*

*cdd* : : *Tn*10). Les transductants ont été sélectionnés sur le milieu décrit ci-dessus supplémenté en tétracycline ce qui a permis d'isoler la souche PAK9 (MG1655 Δ*pyrC* ::Gm, Δ*codA* ::Km, *cdd* ::*Tn*10). La souche PAK9 est auxotrophe pour l'uracile (U), l'uridine (rU) et la 2'-désoxyuridine (dU) et est incapable de croître sur milieu minimum minéral supplémenté en glucose et en cytosine (C), cytidine (rC) ou 2'-deoxycytidine (dC).

## 2- **Mutagenèses**

### 2.1 Variation de la concentration en manganèse lors de la réaction PCR

Les amorces FP23 (5'-CGCCAGGGTTTTCCCAGTCACG) (SEQ ID No 12) et RP23 (5'-AGCGGATAACAATTTCACACAGG) (SEQ ID No 13) ont été utilisés pour amplifier le gène *ntd* cloné dans le plasmide pSU19 selon des conditions standard d'amplification excepté la concentration finale en dNTPs : 20 μM et la concentration en ion $Mn^{2+}$ qui varie de 0 à 0,5 mM final selon les expériences. Les paramètres d'amplification 1 cycle de 5 minutes à 95°C, 40 cycles comportant chacun les trois étapes suivantes : 30 secondes à 95°C, 30 secondes à 53°C, 3 minutes à 72°C, puis un cycles de 10 minutes à 72°C.

### 2.2 Mutagenèse par incorporation d'un analogue de purine dYTP

Différentes réactions d'amplification ont été effectuées en utilisant les oligonucléotides RP23 et FP23 comme amorces, le plasmide pSU19 contenant le gène *ntd* comme ADN matrice. Afin de substituer le dATP, les concentrations suivantes ont été utilisées : dYTP (1mM), dGTP, dCTP et dTTP (200 μM) dATP variant de 1 à 5 μM. Afin de substituer le dGTP, les concentrations suivantes ont été utilisées : dYTP (1 mM), dATP, dCTP et dTTP (200 μM) dGTP variant de 1 à 5 μM. Les paramètres d'amplification sont les mêmes que ceux qui sont décrits ci-dessus la troisième étape exceptée où le temps d'élongation est de 10 minutes. Une seconde réaction d'amplification est ensuite réalisée dans les conditions standard en utilisant 10 μL de la première amplification

## 3- Clonage et sélection

Les produits d'amplification purifiés sont digérés durant 2 heures à 37°C par les enzymes de restriction *BamH*I et *Hind*III. Après migration à 150 V durant 45 min, ils sont purifiés par extraction du gel d'agarose à 1 % à l'aide du kit QIAquick gel extraction (QIAgen).

Le plasmide pSU19 est digéré par les mêmes enzymes et purifié selon la même procédure.

Les ligatures réalisées dans un volume de 20 μL comprennent 15 ng des produits d'amplification, 50 ng de pSU19 digéré par *BamH*I-*Hind*III, 2 μL de tampon de réaction 10x concentré de la T4 ADN ligase et 6 U de T4 ADN ligase. La réaction est effectuée à 16°C durant 18 heures.

Les produits de ligature sont ensuite dialysés sur filtre Millipore (0,05 μm ; 13 mm) pendant 30 min puis utilisés pour transformer la souche PAK9, préparée selon le protocole décrit par Dower et coll (1987), par électroporation.

1 à 5 μL d'ADN ligaturé mélangés à 50 μL de la souche PAK9 dans une cuvette de 2 mm sont soumis à une charge de 2,5 kV. Après une incubation d'une heure à 37°C dans 1 ml de milieu LB supplémenté en uracile (0,3 mM), deux lavages successifs avec 1 ml milieu MS 1x sont effectués.

450 μL de suspension sont étalés sur milieu gélosé minéral glucose supplémenté en Cm, ddU et C. Les boites sont incubées à 37°C durant 4 jours. Les colonies sélectionnées sont ensuite isolées sur le même milieu.

L'ADN plasmidique des colonies isolées est préparé à partir de culture en milieu LB supplémenté en Cm et U. Le séquençage a été effectué par la société MWG-BIOTECH.

## 4- Mesure de l'activité enzymatique des extraits bruts des différents mutants

### 4.1 Préparation des extraits bruts

Les précultures sont obtenues après inoculation d'une colonie isolée dans 5 mL de milieu LB contenant Cm et U pour la souche PAK9 suivie d'une nuit d'incubation sous agitation à 37°C.

Le lendemain, 15 mL de milieu LB Cm et U sont inoculés à une $DO_{600}$ = 0,01. Les cultures sont ensuite incubées à 37°C jusqu'à une DO comprise entre 0,8 et 1.

Les cellules sont ensuite centrifugées à 4 000 rpm pendant 30 minutes à 4°C, le culot est remis en suspension dans 10 ml de tampon phosphate ($Na_2HPO_4$ + $NaH_2PO_4$) à 50 mM (pH = 7,5). Après centrifugation, le culot est remis en suspension dans 1 ml du même tampon. Les cellules, conservées dans de la glace, subissent alors trois cycles de 30secondesde sonication et 30secondesde repos. Après centrifugation à 12000 rpm durant 2 x 15 minutes à 4°C; les surnageants sont récupérés et stockés à -20°C.

**4. 2 Réaction enzymatique**

50 µL d'extrait enzymatique sont additionnés à 200 µL de tampon citrate à 100 mM pH 6,44 en présence de ddU ou dU à 3 mM final et de C à 1 mM final pour la souche PAK9, le tout est incubé à 37°C. L'avancement de la réaction est suivi par CCM (Silice ; éluant : MeOH-$CH_2Cl_2$ (20/80)). Les produits sont révélés sous UV, et les sucres révélés par le réactif de Zücker La disparition des substrats et la formation des produits ont aussi été quantifiés par analyse en HPLC. Les différents produits sont séparés par HPLC analytique avec une colonne phase inverse (100-5C18) en utilisant un débit de 1 ml / min et un gradient linéaire 5-25 % $CH_3CN$ dans un tampon triéthylammonium acétate 10 mM à pH 7,5 pendant 20 min.

**5- Surproduction et purification de la *N*-deoxyribosyltransferase native et du mutant LL7.**

Les oligonucléotides
5'-GATATACATATGCCAAAAAAGACGATCTAC (SEQ ID No 14) et
5'-NNGGATCCTTAGTATACGGCACCTTCGTAGAAGTCG (SEQ ID No 15) ont été utilisés comme amorce dans une réaction d'amplification dans des conditions standard en utilisant le gène *ntd* cloné dans le pSU19 ou le mutant 7 sélectionné précédemment comme ADN matrice. Chaque produit d'amplification a été digéré par les enzymes de restriction *Nde*I et *Bam*HI pendant 2 heures à 37°C, purifié sur gel d'agarose et inséré dans le plasmide pET24a (NOVAGEN) digéré par les mêmes enzymes puis le mélange de ligature utilisé pour transformer la souche β 2033. L'ADN plasmidique des colonies a été préparé et digéré par les enzymes *Nde*I et *Bam*HI. Ceux dont la séquence était correcte ont été utilisés pour transformer la souche BL21 (DE3)/pLYS (NOVAGEN). La surproduction des deux enzymes a été obtenue à partir de cultures de 500 ml de milieu LB supplémenté en Km et Cm. Ces cultures ont été induites à une $DO_{600}$ = 1 par addition d'IPTG (0,4 mM), l'incubation étant poursuivie pendant 2 heures à 37°C.

Les cellules sont ensuite centrifugées 15 minutes à 4000 rpm à 4°C, lavées dans 50 ml de tampon phosphate puis le culot obtenu après centrifugation est conservé une nuit à -20°C. Le culot bactérien remis en suspension dans 20 ml de tampon phosphate est ensuite lysé par un passage à la presse de French à 14000 psi. Le lysat est centrifugé pendant 90 minutes à 50000 rpm. Le surnageant contenant les protéines solubles est ensuite précipité au sulfate d'ammonium (55 % saturation). Le précipité obtenu après centrifugation à 13 900 rpm (20 000 g) pendant 30 minutes à 4°C est remis en suspension dans 5 ml de tampon phosphate 100 mM pH 7,5 puis dialysé durant une nuit à 4°C contre 1e même tampon à pH 6,0. L'extrait enzymatique est ensuite chauffé à 60°C pendant 5 minutes puis replacé immédiatement dans la glace. Les protéines dénaturées sont éliminées par centrifugation à 20 000 rpm pendant 30 minutes à 4°C. La pureté de chaque enzyme est ensuite analysé par gel SDS-PAGE et la concentration déterminée à l'aide du dosage de Bradford en utilisant une gamme de BSA comme standard. La mesure des activités enzymatiques est effectuée comme décrit dans le paragraphe 4.2.

**RESULTATS**

**Test de l'activité des extraits enzymatiques**

L'activité présente dans l'extrait enzymatique préparé est dosée en suivant l'apparition du produit sur CCM. Deux types de réaction sont testés selon que le sucre transféré est un désoxyribose ou un didésoxyribose.

Après 3 heures d'incubation à 37°C, seuls les extraits des mutants catalysent la réaction de transfert

$$ddU + C \rightarrow ddC + U.$$

Ils sont aussi capables comme la souche sauvage de transférer le désoxyribose mais avec une vitesse apparemment plus lente.

D'autre part les réactions

$$ddU + I \rightarrow ddI+U$$

ainsi que

$$ddT+C \rightarrow ddC + T$$

ont aussi donné des résultats positifs avec ces quatre extraits. Ces résultats montrent qu'avec une didésoxypyrimidine comme donneuse, la base acceptrice peut être une purine ou une pyrimidine.

En outre, les extraits provenant des divers mutants semblent se comporter de la même manière. Nous avons

poursuivi l'étude cinétique plus particulièrement sur le mutant pSU-*ntd**C en comparant son activité à celle de la souche sauvage pSU-*ntd*A grâce à des analyses par HPLC.

**Suivi Cinétique des réactions à pH = 7,5**

Nous avons choisi de considérer les réactions (1) et (2) et de prendre dU ou ddU comme donneur et l'hypoxanthine I comme accepteur.

$$(1) \qquad dU + I = dI + U$$

$$(2) \qquad ddU + I = ddI + U$$

Les nucléosides donneurs sont introduits en excès par rapport à la base acceptrice (dans un rapport 3:1) afin de déplacer l'équilibre des réactions dans le sens du transfert du sucre.

L'analyse par HPLC permet de suivre précisément l'avancement de la réaction en fonction du temps. Les produits de réaction sont caractérisés par un temps de rétention t (donné pour un gradient de 0 à 15 d'acétonitrile dans un tampon en 20 minutes). La détection se fait à 254 nm.

| | U | I | dU | dI | ddU | ddI |
|---|---|---|---|---|---|---|
| t (min) | 3,8 | 6,7 | 7,8 | 9,3 | 10,4 | 11,9 |

***Réaction (1):*** **transfert de désoxyribose**

Comme le montre la figure 3, la N-désoxyribosyltransférase NTD II de pSU-*ntd* A catalyse rapidement cet échange: la formation de U et dI est quasi-immédiate. Après une heure à 37°C, plus de 70 % d'hypoxanthine I (réactif limitant) a réagi et on constate la formation de dI. Au bout de quatre heures; il y a eu consommation de 88 % de I introduit initialement en vue de produire dI et U.

La souche mutante est également capable de réaliser l'échange de désoxyribose mais avec une cinétique beaucoup plus lente que la souche sauvage. Une heure à 37°C est nécessaire avant l'apparition des produits: environ 30 % de I ont alors été transformés (figure 4). Même si la réaction s'avère plus lente, l'équilibre est dans les deux cas atteint après douze heures.

***Réaction (2):*** **transfert de didésoxyribose**

Aucune conversion de ddU en ddI n'est obtenue avec les extraits exprimant le gène *ntd* sauvage et ce même au bout de 24 heures à 37°C (figure 5).

Par contre avec les extraits exprimant le gène muté, la N-désoxyribosyltransférase catalyse au bout d'une heure la formation de ddI et de U avec 25 % d'hypoxanthine ayant réagi (figure 6). La figure 6 montre les proportions relatives des réactifs et des produits pour les deux souches après trois heures à 37°C. Dans le cas de l'extrait contenant l'enzyme mutante après la consommation de plus de 50 % de I en quatre heures la réaction diminue. Aucun déplacement de l'équilibre atteint n'est détectée après 24 heures. La réaction se révèle quasiment totale (plus de 90 % de I a réagi afin de synthétiser ddI et U). En revanche avec la souche témoin sont produits seulement 5 % du ddI synthétisable.

L'enzyme NTD* produite par le plasmide pSU-*ntd**C s'avère un excellent catalyseur de la réaction d'échange vis-à-vis de 2',3'-didésoxyribose. Deux aspects capitaux ont été modifiés: la vitesse a nettement augmenté et l'état d'équilibre s'est largement déplacé en faveur du transfert de 2',3'-didésoxyribose. Ainsi la réaction est devenue à la fois rapide et quantitative.

**CONCLUSION**

Différents protocoles de mutagenèse aléatoire ont été testés en vue de modifier la spécificité de la N-désoxyribosyltransférase vis-à-vis des didésoxyriboses. Le premier est basé sur l'altération de la fidélité de la *Taq* polymérase en présence de manganèse, qui dans les conditions testées (0,25 mM) génère un nombre limité de mutations par clone. Le second protocole est basé sur l'utilisation d'un analogue nucléosidique, dYTP, à la place de dGTP qui est décrit pour générer un faible taux de mutations. Il semblerait donc que la conversion d'une activité N-désoxyribosyltransférase en

une activité N-didésoxyribosyltransférase ne requiert qu'une ou deux mutations.

Le nombre de mutants retenus après l'étape de sélection sur boîte reste très faible par rapport au nombre total de mutants générés. Cette observation souligne le pouvoir d'une sélection basée sur des exigences nutritionnelles.

D'autre part les N-désoxyribosyltransférases mutées NTD* exprimées dans la souche Δ*pyrC* Δ*codA* Δ*cdd* peuvent catalyser diverses réactions d'échange telles:

$$ddU + C = ddC + U ;$$

$$ddT + C = ddC + T$$

et

$$ddU + I = ddI + U.$$

Une étude cinétique de cette dernière réaction montre que la vitesse de transfert de didésoxyribose par l'enzyme mutée a été augmentée au moins d'un facteur 10 par rapport à celle de l'enzyme sauvage.

En outre la réaction catalysée par la N-désoxyribosyltransférase NTD* peut être considérée comme rapide et totale avec plus de 90 % du réactif limitant consommé.

L'enzyme mutée est aussi capable de catalyser l'échange de désoxyribose mais avec une vitesse de réaction diminuée de moitié par rapport à celle observée pour l'enzyme native. En outre réitérer une évolution dirigée sur les gènes mutés peut se concevoir. Ainsi, une synthèse enzymatique d'agents antiviraux catalysée par ces enzymes mutées pourrait être envisagée. Cette perspective simplifierait la préparation des didésoxynucléotides, obtenus en général par réduction des désoxynucléotides correspondants.

Le procédé de mutagenèse aléatoire associé à une phase de sélection est applicable à de nombreux gènes codant par exemple pour la synthèse de produits biologiquement intéressants. Il ouvre la porte vers une variabilité prodigieuse de gènes mutés à partir d'un gène unique et peut ainsi permettre d'améliorer une activité déjà existante chez certaines enzymes voire de générer une activité nouvelle.

**<u>REFERENCES</u>**

- Chambers SP, Prior SE, Barstow DA, Minton NP. (1988) The pMTL nic- cloning vectors. I. Improved pUC polylinker regions to facilitate the use of sonicated DNA for nucleotide sequencing. Gene 68:139-49
- Munier H, Gilles AM, Glaser P, Krin E, Danchin A, Sarfati R, Barzu O. (1991) Isolation and characterization of catalytic and calmoduliN-binding domains of Bordetella pertussis adenylate cyclase. Eur J Biochem. 196 : 469-74.
- Dower WJ, Miller JF, Ragsdale CW. (1988) High efficiency transformation of E. coli by high voltage electroporation. Nucleic Acids Res. 16 :6127-45.
- Bartolome B, Jubete Y, Martinez E, de la Cruz F. (1991) Construction and properties of a family of pACYC184-derived cloning vectors compatible with pBR322 and its derivatives. Gene. 102 :75-8
- Carson D.A. & Wasson D.B. (1988) Synthesis of 2',3'-dideoxynucleosides by enzymatic trans-glycosylation. Biochem. Biophys. Res. Comm. 155 : 829-834.
- Fischer, X., Kaun, E. and Genz, ,U. (1990) 2',3'- Dideoxyribofuranosides and process for their production. Ger. Offen. DE 3840160.
- Richaud C, Mengin-Lecreulx D, Pochet S, Johnson EJ, Cohen GN, Marliere P. (1993) Directed evolution of biosynthetic pathways. Recruitment of cysteine thioethers for constructing the cell wall of Escherichia coli. J Biol Chem. 268 :26827-35.


## Revendications

1. Procédé pour faire évoluer une N-désoxyribosyltransférase (DTP) de sorte à obtenir une N-didésoxyribosyltransférase, **caractérisé en ce qu'**il comprend les étapes suivantes :

   a) obtention de mutants DTP* de la séquence codant pour une N-désoxyribosyltransférase (DTP) par mutagenèse aléatoire ;
   b) transformation de cellules d'une bactérie d'*E. coli* Δ*pyrC*, Δ*cod A*, Δ*cdd* déficiente dans la voie métabolique conduisant à l'uracile, comportant un phénotype [U-], avec des vecteurs comportant les acides nucléiques mutés obtenus à l'étape a) codant pour les protéines DTP*, U- signifiant que lesdites cellules sont auxothrophes pour au moins un nucléoside, ledit nucléoside étant le produit de l'action de DTP sur son substrat naturel dR-U ;
   c) mise en culture desdites cellules dans un milieu comprenant un substrat ddR-U ;
   d) sélection des cellules [U- :: DTP*] qui ont survécu à l'étape c) dans lesquelles les protéines DTP* sont capables de réaliser le transfert du didésoxyribose (ddR) d'un didésoxyribonucléoside à un autre nucléoside conduisant à la production du nucléoside N nécessaire pour la survie des cellules.

2. Procédé selon la revendication 1, **caractérisé en ce que** la N-désoxyribosyltransférase (DTP) est une DTP de *Lactobacillus.*

3. Procédé selon la revendication 2, **caractérisé en ce que** la N-désoxyribosyltransférase (DTP) est une DTP de *Lactobacillus leichmannii* de SEQ ID No. 1.

4. N-désoxyribosyltransférase, **caractérisée en ce qu'**elle comprend la séquence SEQ ID No. 2 comportant la mutation G9S, et **en ce qu'**elle présente une activité N-didésoxyribosyltransférase.

5. Acide nucléique comprenant une séquence codant pour la N-didésoxyribosyltransférase de la revendication 4.

6. Acide nucléique comprenant la séquence SEQ ID No. 3.

7. Vecteur d'expression comprenant une séquence codante selon la revendication 5 ou 6.

8. Vecteur selon la revendication 7, **caractérisé en ce que** ladite séquence est fusionnée à un promoteur efficace

dans les cellules eucaryotes et/ou procaryotes.

9. Vecteur selon l'une des revendications 7 et 8, **caractérisé en ce qu'**il s'agit d'un plasmide capable de transformer et de se maintenir chez *E. coli.*

10. Cellule hôte comprenant un vecteur selon l'une des revendications 7 à 9.

11. Utilisation d'une N-didésoxyribosyltransférase selon la revendication 4 pour le transfert d'un didéoxyribose (ddR) d'un didésoxyribonucléoside sur un autre nucléoside.

12. Utilisation selon la revendication 11 pour la préparation d'analogues de nucléosides ou nucléotides possédant des propriétés antitumorales.

13. Utilisation selon la revendication 11 pour la préparation du ddI ou ddC.

14. Procédé de préparation de composés comprenant une étape consistant à mettre en oeuvre une protéine mutée selon l'une des revendications 4 et 5.

15. Procédé selon la revendication 14 pour la préparation d'analogues de nucléosides ou nucléotides utiles pour le traitement du cancer ou de maladies infectieuses, notamment des didésoxyribonucléosides, en particulier le ddC et le ddI.

16. Souche d'*E. coli* PAK 9 de génotype Δ*pyrC* ::Gm, Δ*codA* ::Km, *cdd* ::*Tn*10 déposée à la CNCM sous le numéro d'accession I-2902.

## Claims

1. Method for evolving an N-deoxyribosyltransferase (DTP) so as to obtain an N-deoxyribosyltransferase, **characterized in that** it comprises the following stages:

   a) obtaining DTP* mutants from the sequence coding for an N-deoxyribosyltransferase (DTP) by random mutagenesis;
   b) transformation of cells of an *E. coli* Δ*pyrC,* Δ*cod A,* Δ*cdd* bacterium deficient in the metabolic pathway leading to uracil, containing a phenotype [U-], with vectors containing the mutated nucleic acids obtained in stage a) coding for the DTP* proteins, U- signifying that said cells are auxotrophic for at least one nucleoside, said nucleoside being the product of the action of DTP on its natural substrate dR-U.;
   c) culture of said cells in a medium comprising a ddR-U substrate;
   d) selection of the cells [U-:: DTP*] which have survived stage c) in which the DTP* proteins are capable of carrying out the transfer of the dideoxyribose (ddR) from a dideoxyribonucleoside to another nucleoside leading to the production of the N nucleoside necessary for the survival of the cells.

2. Method according to claim 1, **characterized in that** the N-deoxyribosyltransferase (DTP) is a DTP of *Lactobacillus.*

3. Method according to claim 2, **characterized in that** the N-deoxyribosyltransferase (DTP) is a DTP of *Lactobacillus leichmannii* of SEQ ID No 1.

4. N-deoxyribosyltransferase, **characterized in that** it comprises the sequence SEQ ID No 2 containing the mutation G9S and **in that** it has an N-dideoxyribosyltransferase activity.

5. Nucleic acid comprising a sequence coding for the N-dideoxyribosyltransferase of claim 4.

6. Nucleic acid comprising the sequence SEQ ID No. 3.

7. Expression vector comprising a coding sequence according to claim 5 or 6.

8. Vector according to claim 7, **characterized in that** said sequence is fused to an effective promoter in the eucaryotic and/or procaryotic cells.

9. Vector according to one of claims 7 and 8, **characterized in that** it is a plasmid capable of transforming and remaining in *E. coli.*

10. Host cell comprising a vector according to one of claims 7 to 9.

11. Use of an N-dideoxyribosyltransferase according to claim 4 for the transfer of a dideoxyribose (ddR) from a dideoxyribonucleoside to another nucleoside.

12. Use according to claim 11 for the preparation of nucleoside analogues or nucleotides having anti-tumorous properties.

13. Use according to claim 11 for the preparation of ddl or ddC.

14. Method for the preparation of compounds comprising a stage consisting of utilizing a mutated protein according to one of claims 4 and 5.

15. Method according to claim 14 for the preparation of nucleoside analogues or nucleotides useful for the treatment of cancer or infectious diseases, in particular dideoxyribonucleosides, in particular ddC and ddl.

16. PAK 9 strain of *E. coli* of genotype *∆pyrC:: Gm, ∆codA::Km, cdd:: Tn10* deposited at the CNCM under accession number 1-2902.

**Patentansprüche**

1. Verfahren zur Evolution einer N-Desoxyribosyltransferase (DTP) derart, dass man eine N-Didesoxyribosyltransferase erhält, **dadurch gekennzeichnet, dass** es folgende Schritte umfasst:

   a) Erhalten von Mutanten DTP* der Sequenz, die für eine N-Desoxyribosyltransferase (DTP) kodiert, durch zufallsgemäße Mutagenese;
   b) Transformation von Zellen eines *E. coli ∆pyrC, ∆cod A, ∆cdd*-Bakteriums, das eine Defizienz in dem zu Uracil führenden Stoffwechselweg aufweist und einen [U-]-Phänotyp hat, mit Vektoren, die die im Schritt a) erhaltenen mutierten Nukleinsäuren enthalten, die für die DTP*-Proteine kodieren, wobei U- bedeutet, dass die Zellen für mindestens ein Nukleosid auxotroph sind, wobei das Nukleosid das Produkt der Einwirkung von DTP auf ihr natürliches Substrat dR-U ist;
   c) Kultivieren der Zellen in einem Medium, das ein Substrat ddR-U enthält;
   d) Selektion von [U- : : DTP*]-Zellen, die den Schritt c) überlebt haben, in denen die DTP*-Proteine in der Lage sind, die Übertragung von Didesoxyribose (ddR) von einem Didesoxyribonukleosid auf ein anderes Nukleosid durchzuführen, was zur Herstellung des Nukleosids N führt, das für das Überleben der Zellen notwendig ist.

2. Verfahren nach Anspruch 1, **dadurch gekennzeichnet, dass** die N-Desoxyribosyltransferase (DTP) eine DTP von *Lactobacillus* ist.

3. Verfahren nach Anspruch 2, **dadurch gekennzeichnet, dass** die N-Desoxyribosyltransferase (DTP) eine DTP von *Lactobacillus leichmannii* mit der SEQ ID NR: 1 ist.

4. N-Desoxyribosyltransferase, **dadurch gekennzeichnet, dass** sie die Sequenz SEQ ID NR: 2 mit der Mutation G9S umfasst, und dadurch, dass sie eine N-Didesoxyribosyltransferase-Aktivität besitzt.

5. Nukleinsäure, umfassend eine Sequenz, die für die N-Didesoxyribosyltransferase nach Anspruch 4 kodiert.

6. Nukleinsäure, die die Sequenz SEQ ID NR: 3 umfasst.

7. Expressionsvektor, umfassend eine kodierende Sequenz nach Anspruch 5 oder 6.

8. Vektor nach Anspruch 7, **dadurch gekennzeichnet, dass** die Sequenz mit einem Promotor fusioniert ist, der in Eukaryonten- und/oder Prokaryontenzellen wirksam ist.

9. Vektor nach einem der Ansprüche 7 und 8, **dadurch gekennzeichnet, dass** es sich um ein Plasmid handelt, das

*E. coli* transformieren und darin aufrechterhalten werden kann.

**10.** Wirtszelle, umfassend einen Vektor nach einem der Ansprüche 7 bis 9.

**11.** Verwendung einer N-Didesoxyribosyltransferase nach Anspruch 4 für die Übertragung einer Didesoxyribose (ddR) von einem Didesoxyribonukleosid auf ein anderes Nukleosid.

**12.** Verwendung nach Anspruch 11 zur Herstellung von Analoga von Nukleosiden oder Nukleotiden, die Antitumoreigenschaften besitzen.

**13.** Verwendung nach Anspruch 11 zur Herstellung von ddI oder ddC.

**14.** Verfahren zur Herstellung von Verbindungen, das einen Schritt umfasst, bei dem man ein mutiertes Protein nach einem der Ansprüche 4 und 5 einsetzt.

**15.** Verfahren nach Anspruch 14 zur Herstellung von Analoga von Nukleosiden oder Nukleotiden, die sich zur Behandlung von Krebs oder Infektionskrankheiten eignen, insbesondere von Didesoxyribonukleosiden, besonders ddC und ddI.

**16.** *E. coli*-Stamm PAK 9 mit dem Genotyp Δ*pyrC* : : Gm, Δ*codA* : : Km, *cdd : : Tn10*, der bei der CNCM unter der Zugangsnummer 1-2902 hinterlegt worden ist.

**Figure1a : Schématisation de la voie "*de novo*" de UTP et CTP chez *E. coli***

| | *pyrA* | | *pyrD* | | *pyrH* | | *ndk* | | *pyrG* | |
|---|---|---|---|---|---|---|---|---|---|---|
| $HCO_3^-$ | → | dihydroorotate | → | UMP | → | UDP | → | UTP | → | CTP |
| | *pyrB* | | *pyrE* | | | | | | | |
| | pyrC | | *pyrF* | | | | | | | |

*ndk*: nucléoside diphosphokinase
*pyrA*: carbamoylphosphate synthase
*pyrB*: aspartate carbamoyltransférase
*pyrC*: dihydroorotase
*pyrD*: dihydroorotate oxydase
*pyrE*: orotate phosphoribosyltransférase
*pyrF*: orotidine 5'-phosphate decarboxylase
*pyrG*: CTP synthétase
*pyrH*: UMP kinase

**Figure1b: Voie de recyclage des pyrimidines chez *E. coli***

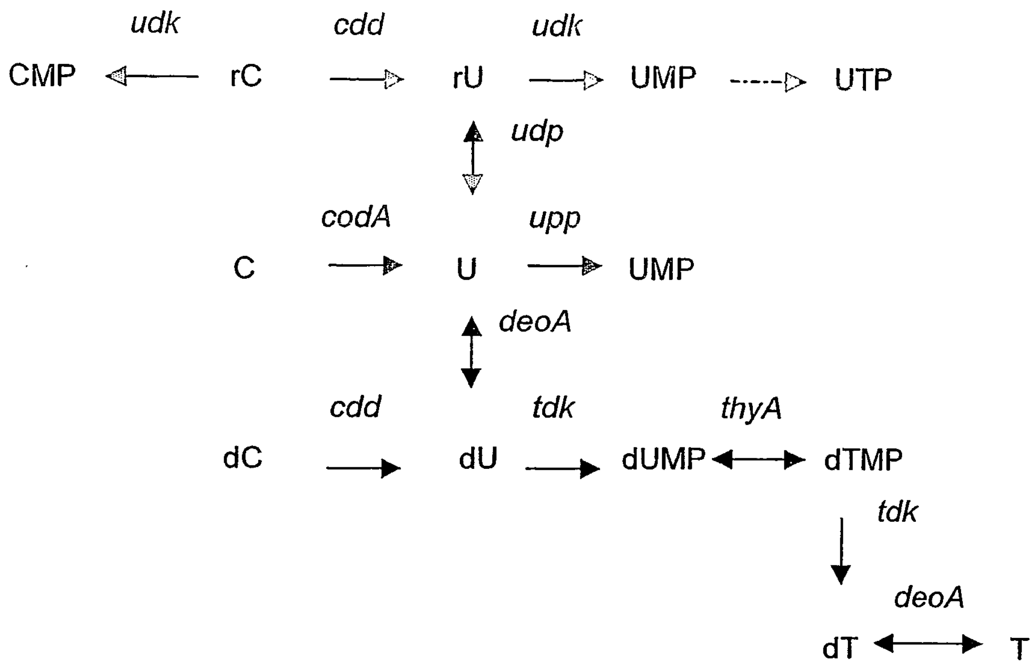


*cdd*: cytidine/désoxycitidine désaminase

*cmk*: CMP/dCMP kinase horylase

*codA*: cytosine désaminase

*deoA*: thymidine phosphorylase

*tdk*: thymidine kinase

*udk*: uridine/cytidine kinase

*udp*: uridine phosphorylase

*upp*: uridine phosphoryltransferase

*thyA*: thymidylate synthase

Les enzymes sont ci-dessus représentées par leurs gènes correspondants.

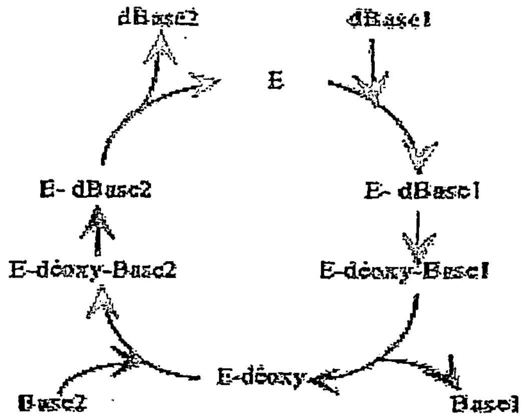


*E-désoxy=complexe enzyme-désoxyribose de la forme:
(E=site actif de l'enzyme)

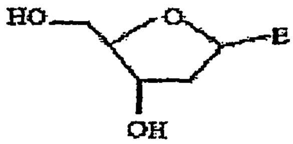

*dBase=désoxyribonucléotide:

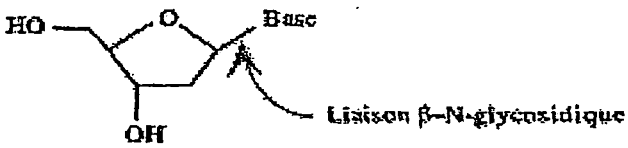


Figure 2

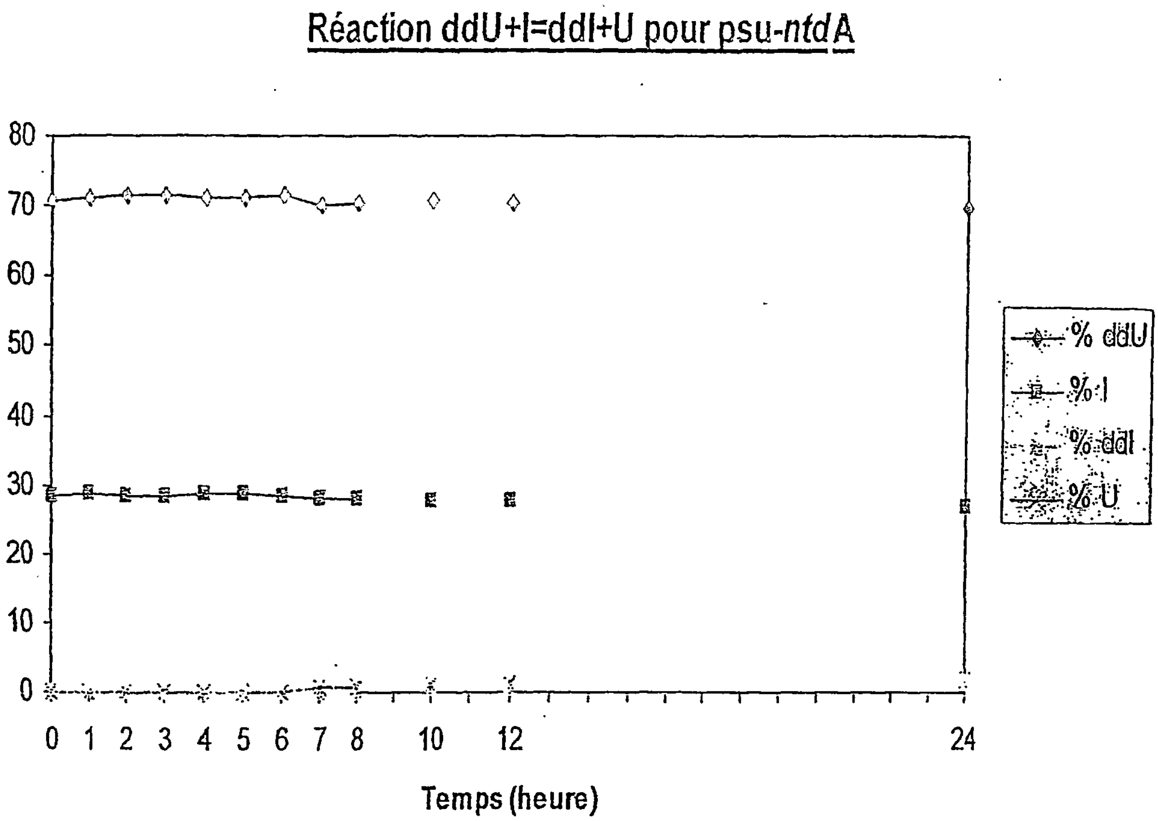
Réaction ddU+I=ddI+U pour psu-ntdA
80
70
60
50
40
30
20
10
0
0 1 2 3 4 5 6 7 8 10 12 24
Temps (heure)
% ddU
% I
% ddI
% U

**Figure 3**

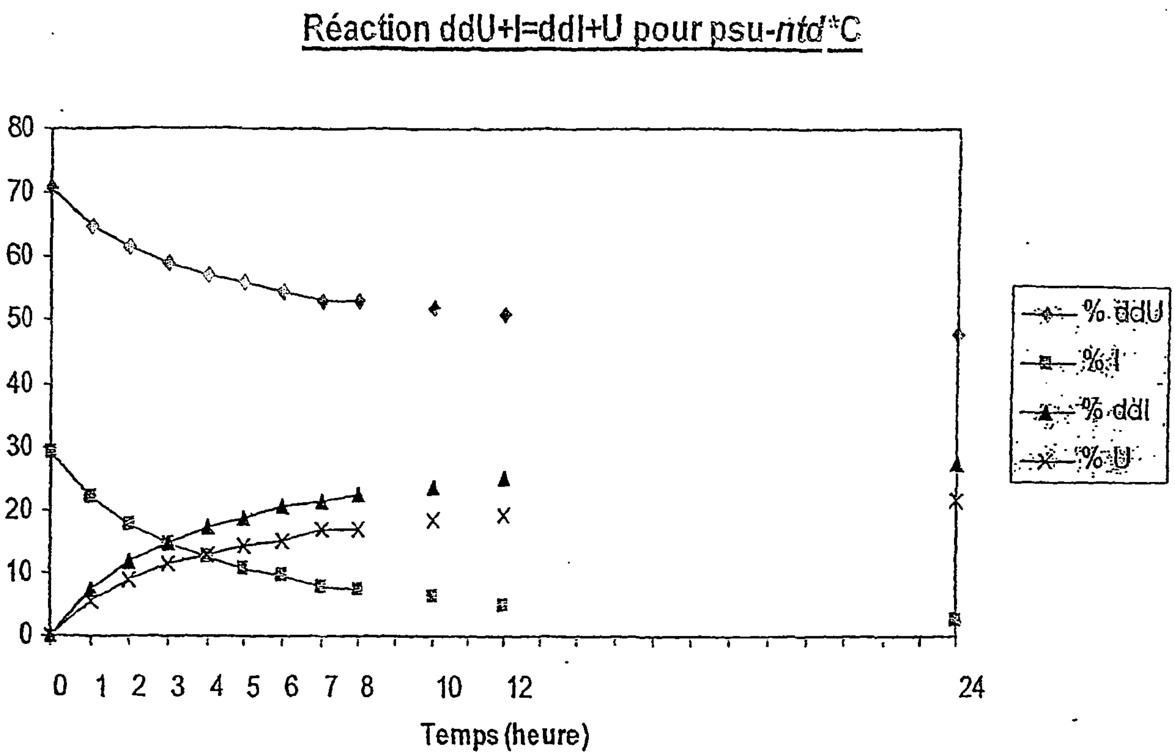
Réaction ddU+I=ddI+U pour psu-ntd*C
80
70
60
50
40
30
20
10
0
0 1 2 3 4 5 6 7 8 10 12 24
Temps (heure)
% ddU
% I
% ddI
% U

**Figure 4**

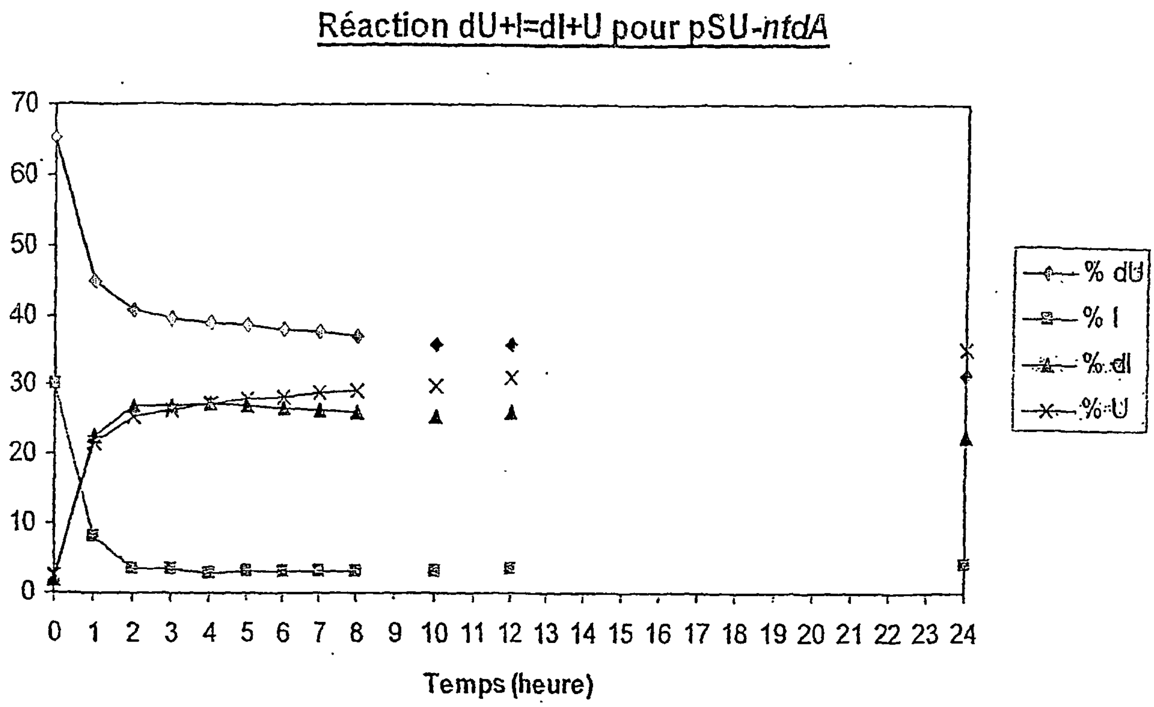
Réaction dU+I=dI+U pour pSU-*ntdA*
70
60
50
40
30
20
10
0
0 1 2 3 4 5 6 7 8 9 10 11 12 13 14 15 16 17 18 19 20 21 22 23 24
Temps (heure)
% dU
% I
% dI
% U

**Figure 5**

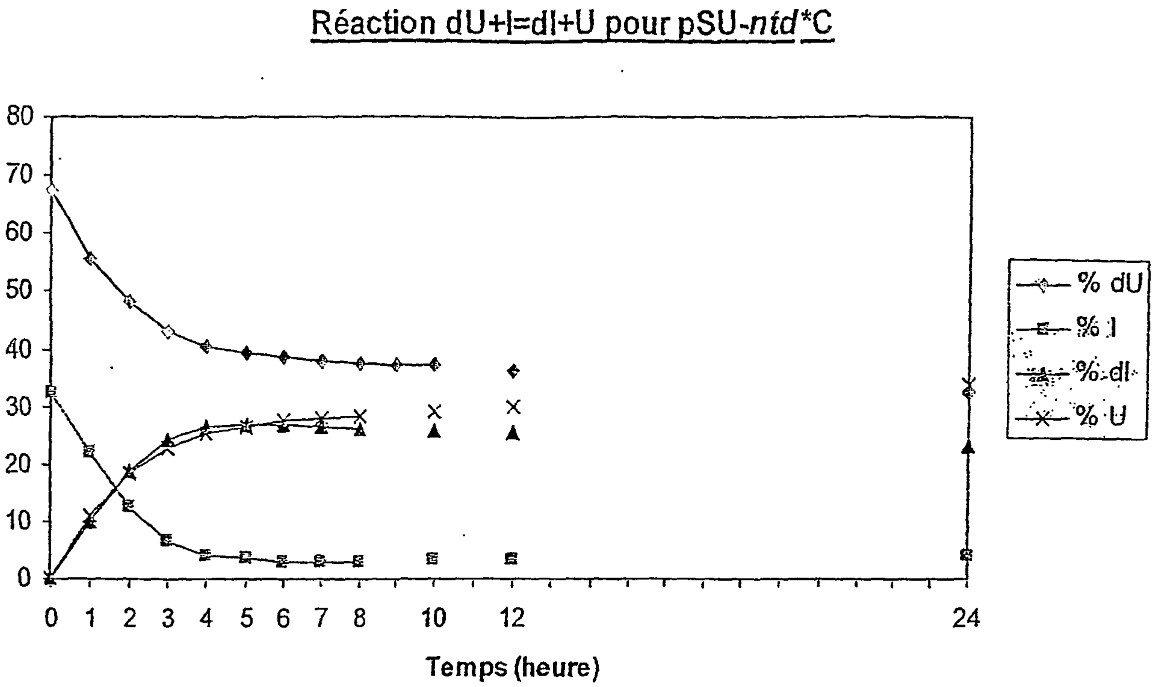
Réaction dU+I=dI+U pour pSU-*ntd**C
80
70
60
50
40
30
20
10
0
0 1 2 3 4 5 6 7 8 10 12 24
Temps (heure)
% dU
% I
% dI
% U

**Figure 6**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 02083892 A
- WO 03025163 A
- US 6323030 B
- US 6177263 B
- WO 0166798 A
- EP 1205547 A
- WO 0196354 A
- DE 3840160
- FR 0303910


### Littérature non-brevet citée dans la description


- **Hall et al.** *Protein Eng.,* 1991, vol. 4, 601
- **Hemsley et al.** *Nucleic Acids Research,* 1989, vol. 17, 6545-6551
- **Ho et al.** *Gene,* 1989, vol. 77, 51-59
- **Hultman et al.** *Nucleic Acids Research,* 1990, vol. 18, 5107-5112
- **Jones et al.** *Nature,* 1990, vol. 344, 793-794
- **Jones et al.** *Biotechniques,* 1992, vol. 12, 528-533
- **Landt et al.** *Gene,* 1990, vol. 96, 125-128
- **Nassal et al.** *Nucleic Acids Research,* 1990, vol. 18, 3077-3078
- **Nelson et al.** *Analytical Biochemistry,* 1989, vol. 180, 147-151
- **Vallette et al.** *Nucleic Acids Research,* 1989, vol. 17, 723-733
- **Watkins et al.** *Biotechniques,* 1993, vol. 15, 700-704
- **Weiner et al.** *Gene,* 1993, vol. 126, 35-41
- **Yao et al.** *PCR Methods and Applications,* 1992, vol. 1, 205-207
- **Weiner et al.** *Gene,* 1994, vol. 151, 1, 9-123
- **Chang, H.K. et al.** Directed Evolution of Comamonas testosterone GZ39 m-hydroxybenzoate hydroxylase for the synthesis of 4-substituted catechols. *Abstracts of the General Meeting of the American Society for Microbiology,* 2003, ISSN 1060-2011
- **Wan L. et al.** In vitro evolution of horse heart myoglobin to increase peroxydase activity. *PNAS,* 1998, vol. 95 (22), 12825-12831
- **Agnete MUNCH-PETERSEN.** Metabolism of nucleotides, nucleosides and nucleobases in microorganisms. Academic Press, 1983
- **Jane R. HANRAHAN ; David W. HUTCHINSON.** The enzymatic synthesis of antiviral agents. *Journal of Biotechnology,* 1992, vol. 23, 193-210
- **Thomas A. KRENITSKY ; George W. KOASALKA ; Joel TUTTLE.** Purine nucleoside synthesis, an efficient method employing nucleoside phosphorylase. *Biochemistry,* 1981, vol. 20, 3165-3621
- **A.R. MUSHEGIAN ; E.V. KOONIN.** Unexpected sequence similarity between nucleosidases and phosphoribosyltransferases of different specificity. *Protein Science,* 1994, vol. 3, 1081-1088
- **José HOLGUIN ; Robert CARDINAUD.** Trans-N-Deoxyribosylase: substrate specific studies. *European Journal of Biochemmistry,* 1975, vol. 54, 515-520
- **José HOLGUIN ; Robert CARDINAUD.** Trans-N-Deoxyribosylase: Purification by affinity chromatography and characterisation. *European Journal of Biochemmistry,* 1975, vol. 54, 505-514
- **C. DANZIN ; Robert CARDINAUD.** Deoxyribosyl transfer catalysis with trans-N-deoxyribosylase. Kinetic studies of purine to purine trans-N-deoxyribosylase. *European Journal of Biochemistry,* 1974, vol. 48, 255-252
- **C. DANZIN ; Robert CARDINAUD.** Deoxyribosyl transfer catalysis with trans-N-deoxyribosylase. Kinetic studies of purine (pyrimidine) to purine (pyrimidine) trans-N-deoxyribosylase. *European Journal of Biochemistry,* 1976, vol. 62, 356-372
- **William J.COOK ; Steven A. SHORT ; Steven E. EALICK.** Crystallization & preliminary X-ray investigation of recombinant Lactobacillus leichmanii nucleoside 2-deoxyribosyltransferase. *Journal of Biological Chemistry,* 1990, vol. 265 (5), 2682-2683
- **Shelly R. ARMSTRONG ; William J.COOK ; Steven A. SHORT ; Steven E. EALICK.** Crystal structures of nucleoside 2-deoxyribosyltransferase in native & ligand-bound forms reval architecture of the active site. *Structure,* 1996, vol. 4 (1), 97-107

- **David J.T. PORTER ; Barbara M. MERRIL ; Steven A. SHORT.** Identification of the active site nucleophile nucleoside 2-deoxyribosyltransferase as glutamic acid 98. *Journal of Biological chemistry,* 1995, vol. 270 (26), 15551-15556
- **D. BETBEDER ; D.W. HUTCHINSON ; A.O. RICHARDS.** The stereoselective enzymatic synthesis of 9-β-D-2',3'-dideoxynucleosides of N(6)-substitued purines. *Antiviral Chem. Chemother,* 1989, vol. 17, 4217-4222
- **H. MITSUYA ; S. BRODER.** Strategies for antiviral therapy in AIDS. *Nature,* 1987, vol. 325, 773-778
- **Chambers SP ; Prior SE ; Barstow DA ; Minton NP.** The pMTL nic- cloning vectors. I. Improved pUC polylinker regions to facilitate the use of sonicated DNA for nucleotide sequencing. *Gene,* 1988, vol. 68, 139-49
- **Munier H ; Gilles AM ; Glaser P ; Krin E ; Danchin A ; Sarfati R ; Barzu O.** Isolation and characterization of catalytic and calmoduliN-binding domains of Bordetella pertussis adenylate cyclase. *Eur J Biochem.,* 1991, vol. 196, 469-74
- **Dower WJ ; Miller JF ; Ragsdale CW.** High efficiency transformation of E. coli by high voltage electroporation. *Nucleic Acids Res.,* 1988, vol. 16, 6127-45
- **Bartolome B ; Jubete Y ; Martinez E ; de la Cruz F.** Construction and properties of a family of pACYC184-derived cloning vectors compatible with pBR322 and its derivatives. *Gene,* 1991, vol. 102, 75-8
- **Carson D.A. ; Wasson D.B.** Synthesis of 2',3'-dideoxynucleosides by enzymatic trans-glycosylation. *Biochem. Biophys. Res. Comm.,* 1988, vol. 155, 829-834
- **Richaud C ; Mengin-Lecreulx D ; Pochet S ; Johnson EJ ; Cohen GN ; Marliere P.** Directed evolution of biosynthetic pathways. Recruitment of cysteine thioethers for constructing the cell wall of Escherichia coli. *J Biol Chem.,* 1993, vol. 268, 26827-35

SEQUENCE LISTING

<110> INSTITUT PASTEUR
CENTRE NATIONAL DE LA RECHERCHE SCIENTIFIQUE

<120> Procédé de modification in vivo de l’activité de synthèse d’un métabolite par modification d’un gène dont l’activité n’est pas l’activité originelle.

<130> BIF 023274 PCT

<140> PCT/US/FR03/xxxxx
<141> 2003-03-28

<150> FR 03 03910
<151> 2003-03-28

<160> 15

<170> PatentIn version 3.1

<210> 1
<211> 474 <212> DNA <213> Lactobacillus leichmannii

<220>
<221> misc_feature
<222> (1)..(474)
<223> Region codante du gène N-déoxyribosyltransférase (dtp)

<400> 1

```
atgccaaaaa agacgatcta cttcggtgcc ggctggttca ctgaccgcca aaacaaagcc     60
tacaaggaag ccatggaagc cctcaaggaa aacccaacga ttgacctgga aaacagctac    120
gttcccctgg acaaccagta caagggtatc cgggttgatg aacacccgga atacctgcat    180
gacaaggttt gggctacggc cacctacaac aacgacttga acgggatcaa gaccaacgac    240
atcatgctgg gtgtctacat ccctgacgaa gaagacgtcg gcctgggcat ggaactgggt    300
tacgccttga gccaaggcaa gtacgtcctt ttggtcatcc cggacgaaga ctacggcaag    360
ccgatcaacc tcatgagctg ggcgtcagc gacaacgtga tcaagatgag ccagctgaag      420
gacttcaact tcaacaagcc gcgcttcgac ttctacgaag gtgccgtata ctaa           474
```

<210> 2
<211> 157
<212> PRT
<213> Lactobacillus leichmannii

<220>
<221> MISC_FEATURE
<222> (1)..(157)
<223> N-déoxyribosyltransférase portant la mutation G9S.

<220>
<221> MISC_FEATURE
<222> (9)..(9)
<223> mutation sérine/glycine

<400> 2

```
Met Pro Lys Lys Thr Ile Tyr Phe Ser Ala Gly Trp Phe Thr Asp Arg
1               5                   10                  15

Gln Asn Lys Ala Tyr Lys Glu Ala Met Glu Ala Leu Lys Glu Asn Pro
            20                  25                  30

Thr Ile Asp Leu Glu Asn Ser Tyr Val Pro Leu Asp Asn Gln Tyr Lys
        35                  40                  45

Gly Ile Arg Val Asp Glu His Pro Glu Tyr Leu His Asp Lys Val Trp
    50                  55                  60

Ala Thr Ala Thr Tyr Asn Asn Asp Leu Asn Gly Ile Lys Thr Asn Asp
65                  70                  75                  80

Ile Met Leu Gly Val Tyr Ile Pro Asp Glu Glu Asp Val Gly Leu Gly
                85                  90                  95

Met Glu Leu Gly Tyr Ala Leu Ser Gln Gly Lys Tyr Val Leu Leu Val
            100                 105                 110

Ile Pro Asp Glu Asp Tyr Gly Lys Pro Ile Asn Leu Met Ser Trp Gly
        115                 120                 125

Val Ser Asp Asn Val Ile Lys Met Ser Gln Leu Lys Asp Phe Asn Phe
    130                 135                 140

Asn Lys Pro Arg Phe Asp Phe Tyr Glu Gly Ala Val Tyr
145                 150                 155
```

<210> 3
<211> 474
<212> DNA
<213> Lactobacillus leichmannii

<220>
<221> misc_feature
<222> (1)..(479)
<223> Séquence codante de la N-déosyribosyltransferase mutée (NTD*).

<400> 3

atgccaaaaa agacgatcta cttcagtgcc ggctggttca ctgaccgcca aaacaaagcc 60
tacaaggaag ccatggaagc cctcaaggaa aacccaacga ttgacctgga aaacagctac 120
gttcccctgg acaaccagta caagggtatc cgggttgatg aacacccgga atacctgcat 180
gacaaggttt gggctacggc cacctacaac aacgacttga acgggatcaa gaccaacgac 240
atcatgctgg gcgtctacat ccctgacgaa gaagacgtcg gcctgggcat ggaactgggt 300
tacgccttga gccaaggcaa gtacgtcctt ttggtcatcc cggacgaaga ctacggcaag 360
ccgatcaacc tcatgagctg gggcgtcagc gacaacgtga tcaagatgag ccagctgaag 420
gacttcaact tcaacaagcc gcgcttcgac ttctacgaag gtgccgtata ctaa 474

<210> 4
<211> 32
<212> DNA
<213> séquence artificielle

<220>
<221> amorce
<222> (1)..(32)
<223> amorce codBL pour l'amplification du gène PyrC

<220>
<221> misc_feature
<222> (1)..(1)
<223> n est un nucleotide comportant une base A, T, C ou G.

<220>
<221> misc_feature
<222> (2)..(2)
<223> n est un nucleotide comportant une base A, T, C ou G.

<220>
<221> misc_feature
<222> (3)..(3)
<223> n est un nucleotide comportant une base A, T, C ou G.

<400> 4
nnncccgggc ttcttgctcg cttctcgttt gg 32

<210> 5
<211> 29
<212> DNA
<213> Séquence artificielle

<220>
<221> amorce
<222> (1)..(29)
<223> amorce cynTR pour amplifier le gène pyrC.

<220>
<221> misc_feature
<222> (1)..(1)
<223> n est un nucleotide comportant une base A, T, C ou G.

<220>
<221> misc_feature
<222> (2)..(2)
<223> n est un nucleotide comportant une base A, T, C ou G.

<400> 5
nnggatccgt ttgaccgtag cgggcgaac 29

<210> 6
<211> 29
<212> DNA
<213> Séquence artificielle

<220>
<221> amorce
<222> (1)..(29)
<223> Amorce codBR permettant de déleter le gène CodA pour la construct ion de la souche PAK9.

<220>
<221> misc_feature
<222> (1)..(29)
<223> n est un nucleotide comportant une base A, T, C ou G.

<400> 6
ngaattctta ttcgacactg ttagcctcc 29

<210> 7
<211> 27
<212> DNA
<213> Séquence artificielle

<220>
<221> amorce
<222> (1)..(27)
<223> Amorce cynTL utilisé pour déleter le gène CodA dans la constructi on de la souche PAK9.

<220>
<221> misc_feature
<222> (1)..(1)
<223> n est un nucleotide comportant une base A, T, C ou G.

<400> 7
ngaattcacg actgggttac agcgagc 27

<210> 8
<211> 35
<212> DNA
<213> Séquence artificielle

<220>
<221> Amorce
<222> (1)..(35)
<223> Amorce ycEL utilisé pour amplifier un fragment d'ADN de E.coli (M G1655) contenant le gène pyrC.

<220>
<221> misc_feature
<222> (1)..(1)
<223> n est un nucleotide comportant une base A, T, C ou G.

<220>
<221> misc_feature
<222> (2)..(2)
<223> n est un nucleotide comportant une base A, T, C ou G.

<220>
<221> misc_feature
<222> (3)..(3)
<223> n est un nucleotide comportant une base A, T, C ou G.

<400> 8
nnncccgggg ccgacctgct ggcccactct gacgg 35

<210> 9
<211> 38
<212> DNA
<213> Lactobacillus leichmannii

<220>
<221> Amorce
<222> (1)..(38)
<223> Amorce dinR utilisé pour amplifier un fragment d'ADN de E.coli (M G1655) contenant le gène pyrC.

<220>
<221> misc_feature
<222> (1) .. (1)
<223> n est un nucleotide comportant une base A, T, C ou G.

<220>
<221> misc_feature
<222> (2)..(2)
<223> n est un nucleotide comportant une base A, T, C ou G.

<400> 9
nnggatcccc cggcggcagc gcctacggaa ccgctgcc 38

<210> 10
<211> 37
<212> DNA
<213> Lactobacillus leichmannii

<220>
<221> Amorce
<222> (1)..(37)
<223> Amorce yceR utilisé pour l'amplification d'ADN plasmidique trnasf ormant lors de la préparation de la souche PAK9.

<220>
<221> Amorce
<222> (1)..(37)
<223> Amorce yceR utilisée pour l'amplification d'ADN plasmidique trnas formant lors de la préparation de la souche PAK9.

<220>
<221> misc_feature
<222> (1)..(1)
<223> Nuléotide comportant une base A, T, C ou G.

<400> 10
ngaattctta atcagtaaat ggaatgacaa tttcgcc 37

<210> 11
<211> 34
<212> DNA
<213> Lactobacillus leichmannii

<220>
<221> Amorce
<222> (1)..(34)
<223> Amorce dinL utilisée pour l'amplification d'ADN plasmidique trnas formant lors de la préparation de la souche PAK9.

<220>
<221> misc_feature
<222> (1)..(1)
<223> Nucléotide comportant une base A, T, C ou G.

<400> 11
ngaattcaaa tcgtagcttc ctgttgtcat tagg 34

<210> 12
<211> 22
<212> DNA
<213> Séquence artificielle

<220>
<221> Amorce
<222> (1)..(22)
<223> Amorce FP23 pour l'amplification du gène ntd.

<400> 12
cgccagggtt ttcccagtca cg 22

<210> 13
<211> 23
<212> DNA
<213> Séquence artificielle

<220>
<221> Amorce
<222> (1)..(23)
<223> Amorce RP23 pour l'amplification du gène ntd.

<400> 13
agcggataac aatttcacac agg 23

<210> 14
<211> 30
<212> DNA
<213> Séquence artificielle

<220>
<221> Amorce
<222> (1)..(30)
<223> Amorce pour l'amplification du gène ntd cloné dans pSU19 ou son mutant.

<400> 14
gatatacata tgccaaaaaa gacgatctac 30

<210> 15
<211> 36
<212> DNA
<213> Séquence artificielle

<220>
<221> Amorce
<222> (1)..(36) <223> Amorce pour l'amplification du gène ntd cloné dans pSU19 ou son mutant.

<220>
<221> misc_feature
<222> (1)..(1)
<223> n est un nucléotide comportant une base A, T, C ou G.

<220>
<221> misc_feature
<222> (2)..(2)
<223> n est un nucléotide comportant une base A, T, C ou G.

<400> 15
nnggatcctt agtatacggc accttcgtag aagtcg 36